(19) Europäisches Patentamt
European Patent Office
Office européen des brevets

(11) Veröffentlichungsnummer: **0 352 230**
**A2**

# (12) EUROPÄISCHE PATENTANMELDUNG

(21) Anmeldenummer: **89810536.6**

(22) Anmeldetag: **13.07.89**

(51) Int. Cl.5: **C 07 H 15/04**
A 01 N 43/16

(30) Priorität: **22.07.88 CH 2809/88**

(43) Veröffentlichungstag der Anmeldung:
**24.01.90 Patentblatt 90/04**

(84) Benannte Vertragsstaaten:
**AT CH DE FR IT LI**

(71) Anmelder: **CIBA-GEIGY AG**
**Klybeckstrasse 141**
**CH-4002 Basel (CH)**

(72) Erfinder: **Ernst, Beat, Dr.**
**Obere Egg 6**
**CH-4312 Magden (CH)**

## (54) Optische Isomere eines Palmitoylglucopyranosids, ein Verfahren zu deren Herstellung und deren Verwendung.

(57) Verbindung der Formel I

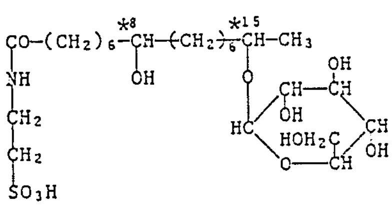

(I)

wobei * die Form ihrer optischen Isomeren (8R,15R), (8R,15S), (8S,15R) und (8S,15S) bedeutet, sowie ihre Alkali- und Erdalkalisalze. Die optischen Isomeren sind über ein Mehrstufenverfahren erhältlich und eignen sich zum Schützen von Kirschen vor dem Befall durch Kirschenfliegen.

EP 0 352 230 A2

## Beschreibung

### Optische Isomere eines Palmitoyl-glucopyranosids, ein Verfahren zu deren Herstellung und deren Verwendung

Die Erfindung betrifft die 4 optischen Isomeren von N-[15-(β-Glucopyranosyl)oxyl-8-hydroxypalmitoyl]-taurin, ein Verfahren zu deren Herstellung und deren Verwendung.

In der EP-A-0 230 397 wird die Isolierung des Markierungsstoffes, den das Weibchen der Krischfliege Rhagoletis cerasi auf der Oberfläche von Kirschen absondert, aus deren Exkrementen beschrieben. Es handelt sich hierbei um das zuvor erwähnte Taurinderivat. Es wird auch darauf hingewiesen, dass in der allgemeinen Formel die vier optischen Isomeren eingeschlossen sind. Es ist jedoch nicht offenbart, welche optischen Isomeren in dem Naturprodukt enthalten sind und welche biologische Aktivität die optischen Isomeren besitzen.

Es bestand somit das Bedürfnis, zum einen für eine wirtschaftliche Anwendung grössere Mengen des Markierungsstoffes über ein synthetisches Verfahren herzustellen und zum anderen gleichzeitig die optischen Isomeren nach einem synthetischen Verfahren herzustellen.

Ein Gegenstand ist eine Verbindung der Formel I

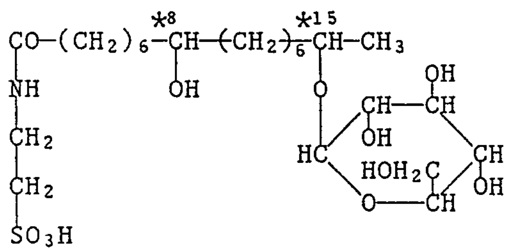

(I)

wobei * die Form ihrer optischen Isomeren (8R,15R), (8R,15S), (8S,15R) und (8S,15S) bedeutet, sowie ihre Alkali- und Erdalkalimetallsalze.

Als Alkali- und Erdalkalimetallsalze kommen z.B. Li-, Na-, K-, Mg- und Ca-Salze in Frage. Bevorzugt sind die Na-, K- und Mg-Salze.

Besonders vorteilhafte biologische Wirkungen zeigen das (8R,15R)- und das (8S,15R)-Isomere.

Ein weiterer Gegenstand ist ein Verfahren zur Herstellung von Verbindungen der Formel I gemäss Anspruch 1, dadurch gekennzeichnet, dass man

a) eine Verbindung der Formel II

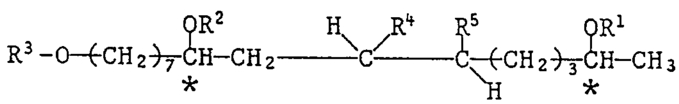

(II),

worin * die optischen Isomeren (R,R), (R,S), (S,R) und (S,S) bedeutet, $R^4$ und $R^5$ je für H oder beide zusammen für eine Bindung stehen und $R^1$, $R^2$ und $R^3$ unabhängig voneinander je eine Schutzgruppe bedeuten, mit in den 2,3,4,6-Stellungen geschützte OH-Gruppen enthaltender α-D-Fluorglucose in Gegenwart von $BF_3$ zu einer Verbindung der Formel III umsetzt,

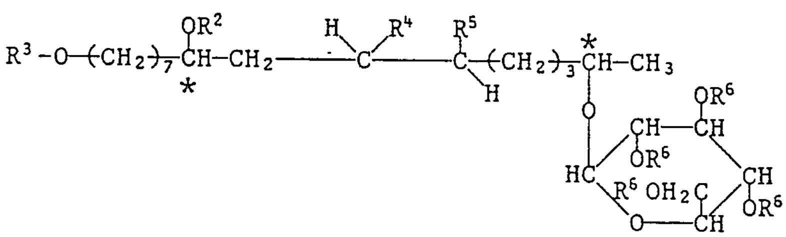

(III),

wobei $R^6$ eine Schutzgruppe bedeutet;

b) danach die Schutzgruppe $R^3$ entfernt und die erhaltene $-CH_2OH$-Gruppe zur Herstellung einer Verbindung der Formel IV

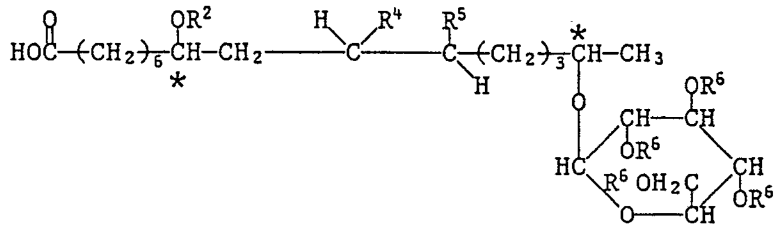

(IV)

oxidiert;

c) die Verbindung der Formel IV in einen zur Knüpfung einer Peptidbindung aktivierten Ester überführt und den aktivierten Ester mit Taurin der Formel $H_2N{-}(CH_2{-})$ $SO_3H$ zu einer Verbindung der Formel V umsetzt,

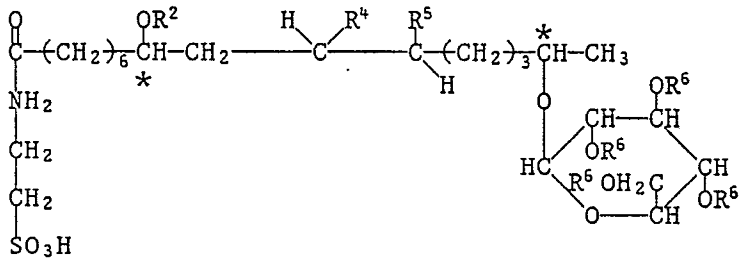

(V),

und darauf

d1) entweder aus den Verbindungen der Formel V, worin $R^4$ und $R^5$ je H bedeuten, die Schutzgruppen $R^2$ und $R^6$ abspaltet und durch H ersetzt; oder

d2) die Verbindungen der Formel V, worin $R^4$ und $R^5$ zusammen eine Bindung bedeuten, hydriert und gleichzeitig die Schutzgruppen $R^2$ und $R^6$ abspaltet und durch H ersetzt.

$R^1$, $R^2$ und $R^3$ können gleiche oder verschiedene Schutzgruppen sein. Hauptsächlich kommen solche Schutzgruppen in Frage, wie sie im allgemeinen in der Synthese von Kohlehydraten verwendet werden. Beispiele für Schutzgruppen sind Trialkylsilyl, ein mit $C_1$-$C_4$-Alkoxy substituiertes Phenyl, unsubstituiertes oder mit $C_1$-$C_4$-Alkyl substituiertes Benzyl, Diphenylmethyl oder Trityl, oder $C_1$-$C_8$-Acyl.

Beispiele für Trialkylsilyl sind Trimethyl-, Triethyl-, Tripropyl-, Tributyl-, Isopropyl-dimethyl-, t-Butyldimethyl, Hexyl-dimethyl-, Octyl-dimethyl- und (1,1,2,2-Tetramethylethyl)-dimethylsilyl.

Bei der Alkoxyphenylgruppe kann es sich um ($C_1$-$C_4$-Alkoxy) phenyl handeln. Die Alkoxygruppe kann linear oder verzweigt und z.B. n-Butoxy, t-Butoxy, n- und i-Propoxy, Ethoxy und besonders Methoxy sein. Einige Beispiele sind p-Methoxyphenyl, 2,4-, 2,6- und 3,4-Dimethoxyphen-1-yl und 3,4,5-Trimethoxyphen-1-yl.

Die Benzyl-, Diphenylmethyl- und Tritylgruppe kann mit linearem oder verzweigtem $C_1$-$C_4$-Alkyl substituiert sein, z.B. mit Methyl, Ethyl, n- und i-Propyl und n- i- und t-Butyl. Beispiele sind p-Methylbenzyl, 2,6-, 2,4-, 3,4-Dimethylbenzyl und 3,4,5-Trimethylbenzyl.

Das Acyl kann ein Rest der Formel R-CO- sein, worin R z.B. H oder lineares oder verzweigtes $C_1$-$C_8$-, besonders $C_1$-$C_6$-Alkyl, $C_5$- oder $C_6$-Cycloalkyl, Phenyl oder Benzyl ist. Beispiele für R sind Methyl, Ethyl und die Isomeren von Propyl, Butyl, Pentyl, Hexyl, Heptyl und Octyl.

In einer Ausführungsform sind $R^1$, $R^2$ und $R^3$ voneinander verschieden und besonders sind $R^1$ Trialkylsilyl, $R^2$ Benzyl, Diphenylmethyl oder Trityl und $R^3$ ist Alkoxy-substituiertes Phenyl oder hat die gleiche Bedeutung wie $R^2$.

Die Schutzgruppen können nach bekannten Methoden eingeführt und abgespalten werden.

Die optischen Isomeren der Formel II können nach allgemein bekannten Verfahren hergestellt werden, wobei eine Herstellungsmethode in den Beispielen 1-43 beschrieben ist. Aus den Verbindungen der Beispiele 40-43 können durch katalytische Hydrierung der Doppelbindung, wobei die Schutzgruppe $R^2$ (Benzyl) ebenfalls abgespalten werden kann, und Wiedereinführung der Schutzgruppe $R^2$ Verbindungen der Formel II hergestellt werden, worin $R^3$ und $R^4$ H bedeuten. Diese Verbindungen der Formel II können analog wie in den Beispielen 44-71 beschrieben zu den optischen Isomeren der Formel I umgesetzt werden.

Die OH-Gruppen der $\alpha$-D-Fluorglucose bei der Verfahrensstufe a) sind bevorzugt mit einer Acylgruppe geschützt, besonders mit Pivaloyl. Die Reaktion wird bevorzugt in einem Halogenkohlenwasserstoff (z.B. $CH_2Cl_2$ oder $CHCl_3$) als Lösungsmittel und in Gegenwart von $BF_3$-Diethyletherat durchgeführt.

Eine bevorzugte Ausführungsform ist dadurch gekennzeichnet, dass in Formel III $R^6$ von $R^2$ verschieden ist, und man $R^6$ abspaltet und danach eine zu $R^2$ identische Schutzgruppe einführt. Insbesondere stellt hierbei $R^2$ Benzyl und $R^6$ Pivaloyl dar.

Die Oxidation bei der Verfahrensstufe b) wird bevorzugt mit einem Cer(IV)-Salz (z.B. Cernitrat) in Gegenwart von Wasser durchgeführt.

Aktivierte Ester zur Knüpfung einer Peptidbindung, wie sie in der Verfahrensstuffe c) verwendet werden, sind für Peptidsynthesen allgemein bekannt und z.B. in R. Geiger, Modern Methods in Protein Chemistry 2

(1985) 399. beschrieben. Bevorzugt sind Ester mit einem N-Hydroxyimidrest in der Estergruppe, z.B. Hydroxysuccinimid-N-oxyl.

Die Methoden der Abspaltung von Schutzgruppen bei der Verfahrensstufe d1) die von der Art der Schutzgruppen abhängen, sind allgemein bekannt und z.B. in T.W. Greene, Protective Groups in Organic Synthesis, John Wiley & Sons, 1981 beschrieben.

Die Hydrierung bei der Verfahrensstufe d2) wird zweckmössig in Gegenwart eines Katalysators, z.B. eines Edelmetallkatalysators wie z.B. Pd oder Pt, durchgeführt.

Ein weiterer Gegenstand sind Verbindungen der Formel II

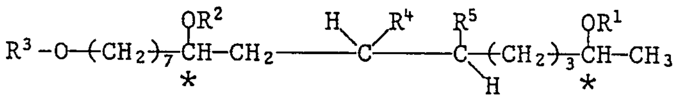 (II),

worin * die optischen Isomeren (R,R), (R,S), (S,R) und (S,S) bedeutet, $R^4$ und $R^5$ je für H oder beide zusammen für eine Bindung stehen und $R^1$, $R^2$ und $R^3$ unabhängig voneinander je eine Schutzgruppe bedeuten. Besonders bevorzugt sind Verbindungen der Formel II, worin $R^4$ und $R^5$ eine Bindung, $R^1$ t-Butyldimethylsilyl, $R^2$ Benzyl und $R^3$ p-Methoxyphenyl sind.

Die optischen Isomeren der Formel I zeigen alle bei der elektrophysiologischen Prüfung biologische Aktivität.

Ein weiterer Gegenstand ist die Verwendung der Verbindungen der Formel I zum Schützen von Kirschen vor dem Befall mit Kirschfliegen.

Die nachfolgenden Beispiele erläutern die Erfindung. $R^1$ ist hierin t-Butyldimethylsilyl, $R^2$ Benzyl, $R^3$ p-Methoxyphenyl und P Pivaloyl.

Beispiel 1:

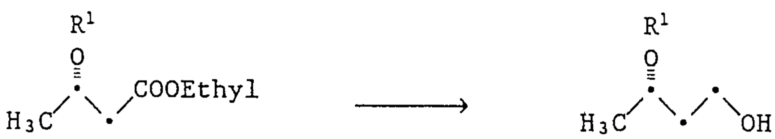

(3S)-1 (3S)-2

15,19 g (61,6 mMol) (3S-1) in 230 ml trockenen THF werden bei 0° C während 2,5 h mit 154 ml einer 1,2 M Lösung (184,9 mMol) von Diisobutylaluminiumhydrid in Toluol versetzt. Zur Aufarbeitung werden nach 4 h 40 ml Methanol und 200 ml K,Na-Tartart (gesättigt) zugegeben und das Reaktionsgemisch 1 h bei Raumtemperatur (RT) gerührt. Anschliessend wird mit Diethylether verdünnt und das Reaktionsgemisch je einmal mit $H_2O$ und Sole gewaschen. Nach dem Trocknen mit $Na_2SO_4$ wird das Lösungsmittel am Rotationsverdampfer (RV) entfernt und der Rückstand bei 16 mbar/105-106° C destilliert. Dabei werden 9,29 g (74 %) (3S)-2 erhalten.

$[\alpha]_D^{25} = +30,3°$ (c = 2,220, Ethanol).

Beispiel 2:

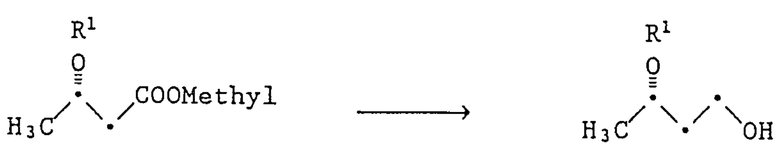

(3R)-1 (3R)-2

Analog zu Beispiel 1 werden ausgehend von 22,54 g (97 mMol) (3R)-1 16,01 g (81 %) (3R)-2 erhalten.

$[\alpha]_D^{25} = -30,7°$ (c = 1,467, $CHCl_3$).

Beispiel 3:

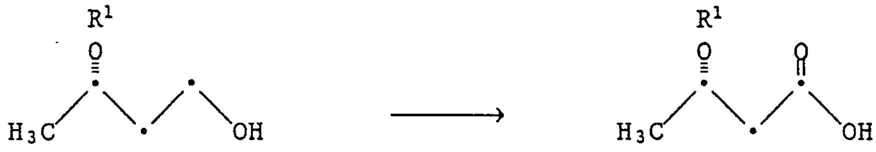

(3S)-2 (3S)-3

Zu 10,8 g (85,2 mMol) Oxalylchlorid in 150 ml Methylenchlorid bei -70°C werden während 10 min. 12,1 ml (170,4 mMol) Dimethylsulfoxid (DMSO) getropft. Anschliessend wird während 10 min. bei -70°C nachgerührt. Dann werden 8,702 g (42,6 mMol) (3S)-2 in 30 ml Methylenchlorid über einen Zeitraum von 35 min. bei -70°C zugetropft und anschliessend 30 min. nachgerührt. Schliesslich werden 59,3 ml (0,426 Mol) Triethylamin während 75 min. bei -70°C zugetropft, das Reaktionsgemisch auf Raumtemperatur erwärmt und in Methylenchlorid aufgenommen. Nach je einmaligem Waschen der organischen Phase mit Wasser, HCl 1N, $NaHCO_3$ (gesättigt) und Sole wird mit $Na_2SO_4$ getrocknet und das Lösungsmittel am Rotationsverdampfer entfernt. Der Rückstand (8,6 g) wird ohne weitere Reinigung in Beispiel 5 eingesetzt.

Beispiel 4:

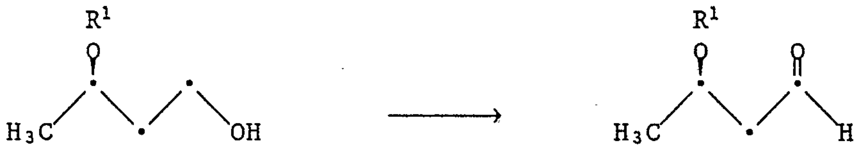

(3R)-2 (3R)-3

Analog zu Beispiel 3 werden ausgehend von 16,01 g (78,4 mMol) (3R)-2 15,6 g (3R)-3 erhalten.
NMR (60 MHz, $CDCl_3$); 9,60 (t, J = 2.0, H-C(1)); 4,27 (q, J = 6, H-C(3)); 2,44 (dd, J = 6.0, 1.0, 2H-C(2)); 1,23 (d, J = 6, $CH_3$); 0.89 (s, $C(CH_3)_3$); 0,08 (s, $2CH_3$).

Beispiel 5:

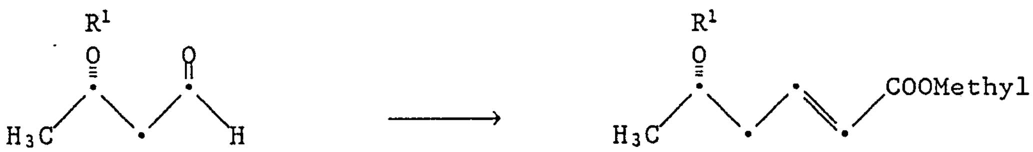

(3S)-3 (5S)-4

8,6 g (42,5 mMol) (3S)-3 und 18,5 g (55,2 mMol) Methoxycarbonylmethyltriphenylphosphoran in 60 ml Methylenchlorid werden 13 h bei Raumtemperatur gerührt. Nach Abdestillieren des Lösungsmittels wird der Rückstand an Kieselgel (Petroleumbenzin 40-60°C/Essigester = 29:1) chromatographiert. Dabei wurden 8,77 g (80 %) (5S)-4 erhalten.
$[\alpha]_D^{25}$ = 19,1° (c = 2,321, Ethanol).

Beispiel 6:

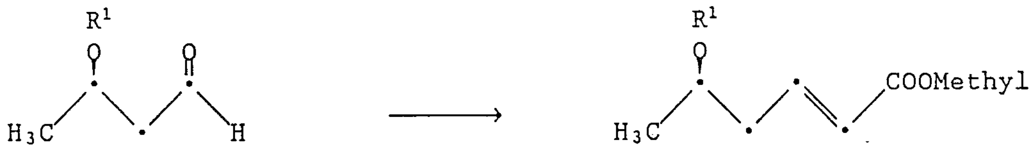

(3R)-3 (5R)-4

Analog zu Beispiel 5 wurden ausgehend von 14,32 g (70,8 mMol) (3R)-3 16,29 g (89 %) (5R)-4 erhalten. $[\alpha]_D^{25}$ = -18,8° (c = 1,215, Ethanol).

Beispiel 7:

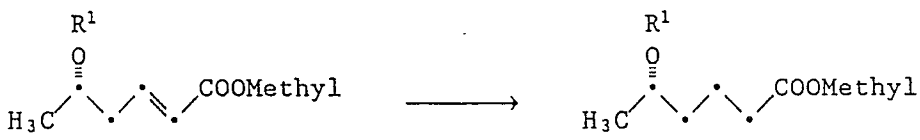

(5S)-4 (5S)-5

8,455 g (32,7 mMol) (5S)-4 und 4,2 g 5 % Pd/$BaSO_4$ in 170 ml Essigester werden bei 22°C während 1 h mit $H_2$ hydriert. Dann wird das Reaktionsgemisch an Celite filtriert und das Lösungsmittel abdestilliert. $[\alpha]_D^{25}$ = +12,9° (c = 1,565, $CHCl_3$).

Beispiel 8:

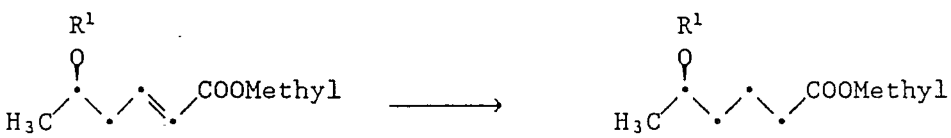

(5R)-4 (5R)-5

Analog zu Beispiel 7 werden ausgehend von 16,29 g (63,03 mMol) (5R)-4 15,34 g (94 %) (5R)-5 erhalten. $[\alpha]_D^{25}$ = -16,5° (c = 1,883, Ethanol).

Beispiel 9:

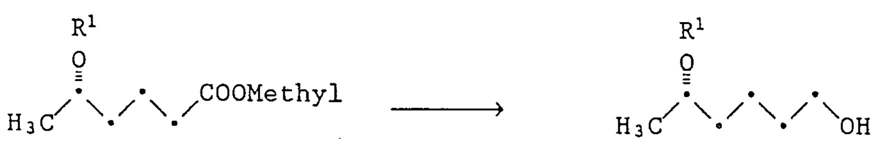

(5S)-5 (5S)-6

Analog zu Beispiel 1 werden 8,12 g (31,2 mMol) (5S)-5 in 100 ml trockenem Toluol mit 65 ml einer 1,2 M Lösung (78 mMol) Diisobutylaluminiumhydrid in Toluol zu 6,68 g (92 %) (5S)-6 reduziert. $[\alpha]_D^{25}$ = +16,2° (c = 1,392, Ethanol).

Beispiel 10:

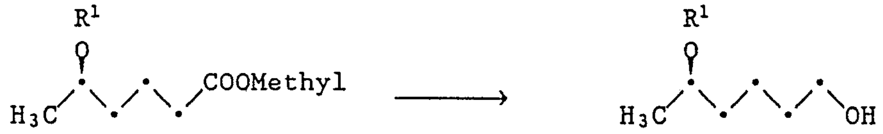

(5R)-5 (5R)-6

Analog zu Beispiel 9 werden ausgehend von 12,43 g (47,7 mMol) (5R)-5 10,05 g (91 %) (5R)-6 erhalten. $[\alpha]_D^{25}$ = -16,5° (c = 0,805, Ethanol).

Beispiel 11:

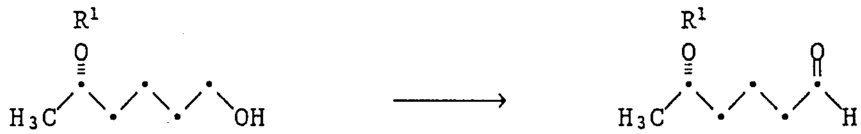

(5S)-6 (5S)-7

Analog zu Beispiel 3 werden 6,0 g (25,8 mMol) (5S)-6 mit 4,5 ml (51,6 mMol) Oxalylchlorid, 8 ml (0,103 M) DMSO und 36 ml (0,258 M) Triethylamin oxidiert. Das Rohprodukt wird an Kieselgel (Petroleumbenzin/Essigester = 29:19) chromatographiert. Dabei werden 5,366 g (90 %) (5S)-7 erhalten.
$[\alpha]_D^{25}$ = +16,9° (c = 1,069, $CHCl_3$).

Beispiel 12:

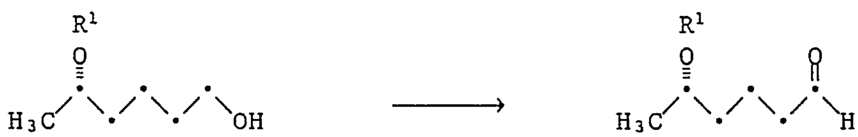

(5R)-6 (5R)-7

Analog zu Beispiel 11 werden ausgehend von 5,94 g (25,56 mMol) (5R)-6 5,41 g (92 %) (5R)-7 erhalten. $[\alpha]_D^{25}$ = -17,4° (c = 1,107, $CHCl_3$).

Beispiel 13:

$CH_3OOC(CH_2)_7OH \longrightarrow CH_3OOC(CH_2)_7OR^3$

8 9

8,0 g (45,9 mMol) 8, 17,1 g (137,7 mMol) Monomethylether von Hydrochinon, 15,65 g (59,7 mMol) Triphenylphosphin und 10,4 g (59,7 mMol) Azodicarbonsäurediethylester in 80 ml Tetrahydrofuran (THF) werden im Bombenrohr 1 h bei 80°C gerührt. Anschliessend wird das Reaktionsgemisch eingeengt und an Kieselgel (n-Hexan/Diethyläther = 9:1) chromatographiert. Dabei wurden 8,7 g (68 %) 9 erhalten.
NMR (60 MHZ, $CDCl_3$): 6,60 (m, 4H); 3,77 (t, J = 6,0, 2H-C(8)); 3,63 (s, $OCH_3$); 3,52 (s, $OCH_3$); 2,22 (m, 2H-C(2)); 1,9-1,0 (m, 10H).

Beispiel 14:

$CH_3OOC(CH_2)_7OR^3 \longrightarrow HO(CH_2)_8OR^3$

9 10

In 3,74 g (98,62 mMol) Lithiumaluminiumhydrid in 150 ml Diethylether bei 0° C werden während 1 h 23,04 g (82,18 mMol) 9 in 100 ml Diethylether getropft. Anschliessend wird das Reaktionsgemisch 1 h am Rückfluss erhitzt. Zur Aufarbeitung wird auf 0° C abgekühlt, langsam mit 4 ml $H_2O$, 12 ml NaOH 15%ig und 12 ml $H_2O$ versetzt, 30 min. am Rückfluss erhitzt und an Celite filtriert. Der nach Abdampfen des Lösungsmittel am Rotationsverdampfer erhaltene Rückstand wird aus Hexan/Diisopropylether umkristallisiert. Dabei wurden 18,6 g (90 %) 10 erhalten.
Smp: 66-68° C.
NMR (300 MHZ, $CDCl_3$): 6,83 (m, 4H); 3,91 (t, J = 6.0, 2H-C(1)); 3,77 (s, $OCH_3$); 3,65 (q, J = 6,0, 2H-C(8)); 1,83-1,70 (m, 2H); 1,57-1,28 (m, 10H).

Beispiel 15:

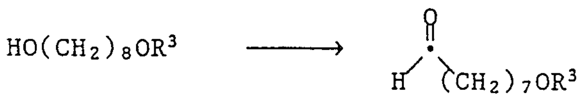

10 11

Analog zu Beispiel 3 werden 500 ml (2,013 mMol) 10 mit 351 µl (4,027 mMol) Oxalylchlorid, 572 µl (8,054 mMol) DMSO und 2,8 ml (20,135 mMol) Triethylamin oxidiert. Das Rohprodukt wird an Kieselgel (n-Hexan/Ether = 6:1) chromatographiert. Dabei werden 465 mg (92 %) 11 erhalten.
NMR (250 MHz, $CDCl_3$); 9,76 (t, J = 2,0, H-C(1)); 6,82 (m, 4H); 3,90 (t, J = 6,0, 2H-C(8)); 3,76 (s, $OCH_3$); 2,42 (txd, J = 7,0, J = 2,0, 2H-C(2)); 1,86-1,26 (m, 10H).

Beispiel 16

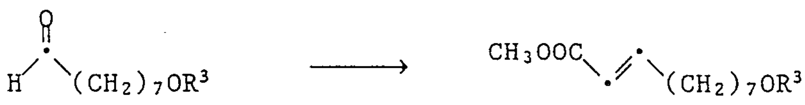

11 12

Analog zu Beispiel 5 werden 2,50 g (8,51 mMol) 11 mit 3,70 g (11,06 mMol) Methoxycarbonylmethyltriphenylphosphoran umgesetzt. Die Chromatographie des Rohproduktes an Kieselgel (Petroleumbenzin 40-60° C/Essigester = 19:1) liefert 195 mg (7 %) Z-12 und 2,353 g (90 %) E-12.
Z-12: NMR (250 MHz, $CDCl_3$): 6,84 (m, 4H); 6,24 (dxt, J = 11,0, J = 2,0, H-C(3)); 5,79 (dxt, J = 11,0, J = 8,0, H-C(2)); 3,88 (t, J = 7,0, 2H-C(10)); 3,75 (s, $OCH_3$); 3,69 (s, $OCH_3$); 2,66 (m, 2H-C(4)); 1,76 (m, 2H); 1,60-1,30 (m, 8H).
E-12: NMR (300 MHz, $CDCl_3$): 6,97 (dxt, J = 15,0, J = 8,0, H-C(3)); 6,83 (m, 4H); 5,82 (dxt, J = 15,0, J = 1,5, H-C(2)); 3,88 (t, J = 7,0, 2H-C(10)); 3,77 (s, $OCH_3$); 3,72 (s, $OCH_3$); 2,20 (m, 2H-C(4)); 1,75 (m, 2H); 1.55-1,20 (m, 8H).

Beispiel 17:

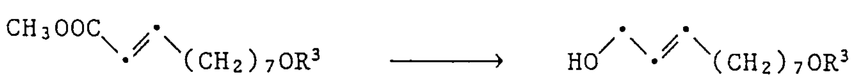

E-12 13

Analog Beispiel 1 werden 5,17 g (16,90 mMol) E-12 in 75 ml trockenem THF mit 42,2 ml einer 1,2 M Lösung (50,6 mMol) Diisobutylaluminiumhydrid in Toluol zu 4,22 g (90 %) 13 reduziert.
Smp. (Umkristallisation aus n-Hexan/Diethylether): 57-58° C.

Beispiel 18:

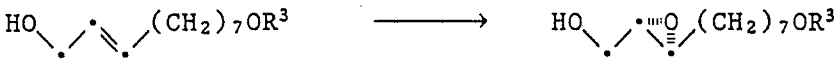

13 (2S,3S)-14

Eine Lösung von 283 µl (1 mMol) Tetraisopropyl-ortho-titanat in 20 ml Methylenchlorid wird bei -68°C nacheinander mit 200 µl (1,2 mMol) L(+)-Weinsäure-diethylester, 500 mg (1,8 mMol) 13 und einer auf -20°C gekühlten, 2,52 M wasserfreien Lösung von tert.Butylhydroperoxid in Toluol versetzt. Anschliessend wird das Reaktionsgemisch über einen Zeitraum von 2,5 h auf 0°C erwärmt und zu einer Lösung von 900 mg (3,23 mMol) Eisen(II)sulfatheptahydrat und 360 mg (1,75 mMol) L(+)-Weinsäure in 10 ml destilliertem $H_2O$ gegeben. Nach Abklingen der exothermen Reaktion wird das Gemisch bei Raumtemperatur während 30 min. gerührt, die wässrige Phase abgetrennt und zweimal mit Diethylether extrahiert. Die vereinigten organischen Phasen werden mit $Na_2SO_4$ getrocknet und am Rotationsverdampfer eingeengt. Der Rückstand wird in 25 ml Diethylether gelöst, auf 3°C gekühlt und mit einer auf 3°C gekühlten Lösung von 150 mg (3,75 mMol) NaOH in 10 ml Sole versetzt. Das resultierende Zweiphasengemisch wird 1 h im Eiswasserbad nachgerührt und anschliessend im Scheidetrichter getrennt. Nochmalige Extraktion der wässrigen Phase mit Diethylether, Trocknen der vereinigten organischen Phasen mit $Na_2SO_4$ und Einengen derselben am Rotationsverdampfer liefert 470 mg rohes (2S, 3S)-14. Chromatographie an Kieselgel (Petroleumbenzin 40-60°C/Essigester = 1:1) ergibt 389 mg (74 %). Nach Umkristallisation aus n-Hexan/Diisopropylether beträgt der Schmelzpunkt 68°C.$[\alpha]_D^{25}$ = -17,4° (c = 0,933, Ethanol), (≧ 97 % ee, anhand des Mosher-Derivates bestimmt).
NMR (250 MHz, $CDCl_3$): 6,81 (m, 4H); 3,90 (t, J = 7, 2H-C(10)); 3,98-3,84 (m, 1H), 3,76 (s, $OCH_3$); 3,66-3,54 (m, 1H); 2,88-3,0 (m, 2H); 1,84-1,66 (m, 2H); 1,66-1,28 (m, 10H).

Beispiel 19:

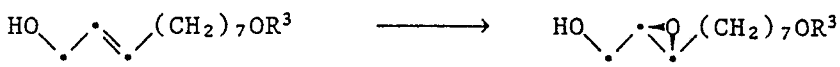

13 (2R,3R)-14

Analog zu Beispiel 18, aber mit D(-)-Weinsäurediethylester, werden aus 4,22 g (15,16 mMol) 13 nach Umkristallisation aus n-Hexan/Diisopropylether 3,88 g (87 %) (2R,3R)-14 erhalten.
Smp.: 68°C;
$[\alpha]_D^{25}$ = +17,5° (c = 0,316, Ethanol; ≧ 97 % ee, anhand des Mosher-Derivates bestimmt).

Beispiel 20:

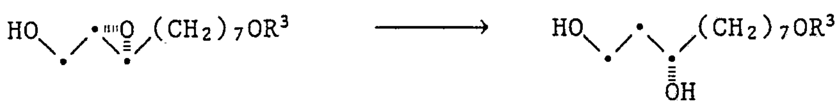

(2S,3S)-14 (3S)-15

In 4,81 g (16,30 mMol) (2S,3S)-14 in 150 ml Toluol werden bei Raumtemperatur 14 ml einer 3,5 M Lösung (49 mMol) von Natrium-dihydro-bis-(2-methoxyethoxy)-aluminat in Toluol während 30 min. getropft. Zur Aufarbeitung wird das Reaktionsgemisch auf 0°C gekühlt, mit 4 ml Methanol und 200 ml einer 1 M Lösung von K-Na-tartrat versetzt und 2 h bei Raumtemperatur nachgerührt. Nach Filtration an Celite wird die wässerige Phase abgetrennt und zweimal mit Essigsäureethylester extrahiert. Die organischen Phasen werden je einmal mit $H_2O$ und Sole gewaschen, mit $Na_2SO_4$ getrocknet und am Rotationsverdampfer eingeengt. Durch Umkristallisation des Rückstandes aus Diisopropylether werden 4,49 g (93 %) (3S)-15 erhalten.
Smp.: 94°C; $[\alpha]_D^{25}$ = +1,6° (c = 1,071, Ethanol).

Beispiel 21:

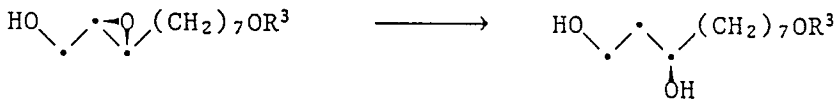

(2R,3R)-14 (3R)-15

Analog Beispiel 20 werden aus 3,35 g (11,38 mMol) (2R,3R)-14 nach Umkristallisation aus Diisopropylether 3,06 g (91 %) (3R)-15 erhalten.
Smp. 95°C; $[\alpha]_D^{25}$ = -2,2° (c = 1,088 Ethanol).

Beispiel 22:

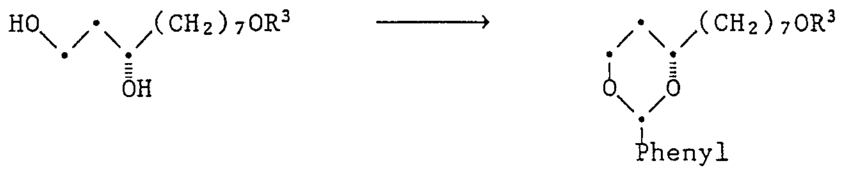

(3S)-15 (3S)-16

4,49 g (15,15 mMol) (3S)-15, 6,13 ml (60,6 mMol) Benzaldehyd und 3 Tropfen konzentrierte $H_2SO_4$ werden in 50 ml DMSO 40 h bei Raumtemperatur gerührt. Zur Aufarbeitung wird das Reaktionsgemisch mit Diethylether verdünnt und je einmal mit gesättigter $NaHCO_3$ und Sole gewaschen. Nach dem Trocknen mit $Na_2SO_4$ wird die Lösung am Rotationsverdampfer eingeengt. Der Rückstand wird an Kieselgel (Petroleumbenzin 40-60°C/Essigester 29:1) chromatographiert. Dabei werden 5,77 g (quantitativ) (3S)-16 erhalten.
$[\alpha]_D^{25}$ = -19,2° (c = 4,431, Ethanol).

Beispiel 23:

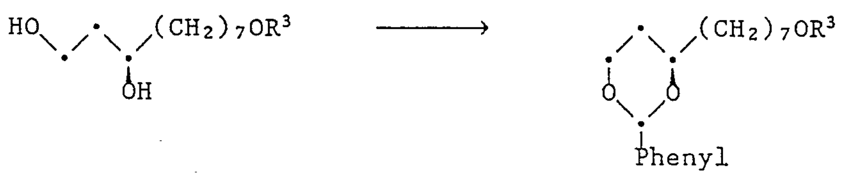

(3R)-15 (3R)-16

Analog Beispiel 22 werden aus 3,05 g (10,29 mMol) (3R)-15 3,79 g (97 %) (3R)-16 erhalten.
$[\alpha]_D^{25}$ = +19,4° (c = 1,286, Ethanol).

Beispiel 24:

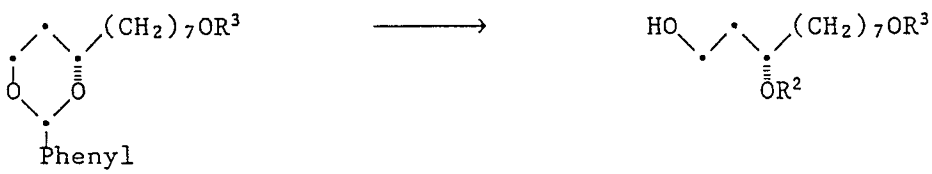

(3S)-16 (3S)-17

5,5 g (14,3 mMol) (3S)-16 in 100 ml n-Hexan werden bei 70°C während 45 min. mit 71,5 ml einer 1 M Lösung von Diisobutylaluminiumhydrid (71,5 mMol) in n-Hexan versetzt. Nach beendetem Zutropfen wird das Reaktionsgemisch auf Raumtemperatur gekühlt, mit 5 ml Methanol und 210 ml einer 1 M Lösung von K-Na-tartrat versetzt und 75 min. nachgerührt. Nach Filtration an Celite wird die wässrige Phase abgetrennt und zweimal mit Diethylether extrahiert. Die organischen Phasen werden je einmal mit $H_2O$ und Sole

gewaschen, mit $Na_2SO_4$ getrocknet und am Rotationsverdampfer eingeengt. Der Rückstand wird an Kieselgel (Petroleumbenzin/Essigester = 3:1) chromatographiert. Dabei werden 4,97 g (90 %) (3S)-17 erhalten. $[\alpha]_D^{25}$ = +5,3° (c = 1,513, Ethanol).

Beispiel 25:

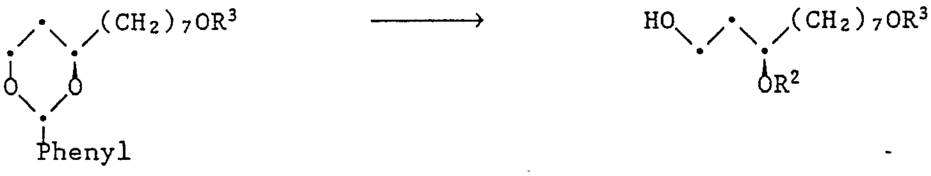

(3R)-16 (3R)-17

Analog Beispiel 24 werden aus 3,72 g (9,7 mMol) (3R)-16 3,46 g (92 %) (3R)-17 erhalten. $[\alpha]_D^{25}$ = -4,9° (c = 1,747, Ethanol).

Beispiel 26:

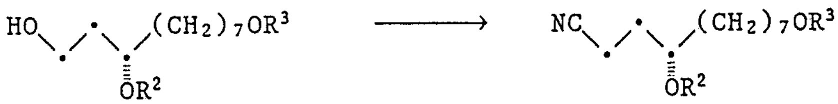

(3S)-17 (3S)-18

15,0 g (38,8 mMol) (3S)-17 in 100 ml Benzol werden bei Raumtemperatur mit 6,34 ml (44,6 mMol) 1-Brom-N,N,2-trimethylpropenylamin versetzt und 1 h gerührt. Anschliessend wird das Benzol bei Normaldruck abdestilliert, 3,8 g (77,6 mMol) NaCN und 100 ml Dimethylformamid (DMF) zugegeben und 40 h bei 130°C gerührt. Zur Aufarbeitung wird mit Diethylether verdünnt, zweimal mit $H_2O$ und einmal mit Sole gewaschen, mit $Na_2SO_4$ getrocknet und am Rotationsverdampfer eingeengt. Der Rückstand wird an Kieselgel (Petroleumbenzin/Essigester = 6:1) chromatographiert. Dabei werden 14,07 (92 %) (3S)-18 erhalten. $[\alpha]_D^{25}$ = +36,5° (c = 0,825, $CHCl_3$).

Beispiel 27:

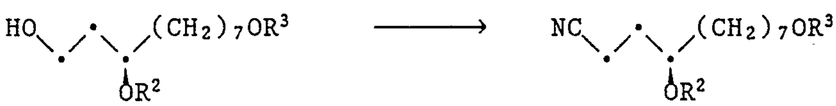

(3R)-17 (3R)-18

Analog zu Beispiel 26 werden ausgehend von 14,305 g (37,0 mMol) (3R)-17 12,692 g (87 %) (3R)-18 erhalten.
$[\alpha]_D^{25}$ = -37,7° (c = 0,795, $CHCl_3$).

Beispiel 28:

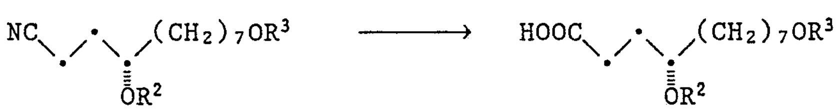

(3S)-18 (4S)-19

14,07 g (35,6 mMol) (3S)-18 und 4,3 g (106,9 mMol) NaOH in 100 ml Ethylenglykol werden 17 h bei 180°C gerührt. Zur Aufarbeitung wird das Reaktionsgemisch mit $H_2O$ verdünnt und zweimal mit Diethylether gewaschen. Anschliessend wird die $H_2O$-Phase mit HCl 2N auf pH 1 angesäuert und drei Mal mit Diethylether

extrahiert. Die organische Phase wird mit $Na_2SO_4$ getrocknet und am Rotationsverdampfer eingeengt. Chromatographie des Rückstandes an Kieselgel ($CHCl_3/CH_3OH$ = 19:1) ergibt 13,65 g (92 %) (3S)-19.
NMR (300 MHz, $CDCl_3$): 7,37-7,22 (m, 5H); 6,82 (m, 4H); 4,53 und 4,46 (AB, J = 12, $CH_2Ph$); 3,89 (t, J = 7, 2H-C(11); 3,77 (s, $OCH_3$); 3,44 (m, H-C(4)); 2,45 (t, J = 7, 2H-C(2)); 2,0-1,23 (m, 14H).

Beispiel 29:

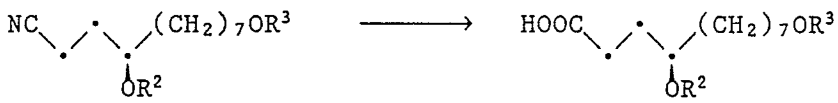

(3R)-18 (4R)-19

Analog Beispiel 28 werden ausgehend von 12,45 g (31,5 mMol) (3R)-18 12,194 g (94 %) (3R)-19 erhalten. $[\alpha]_D^{25}$ = -12,5° (c = 0,971, $CHCl_3$).

Beispiel 30:

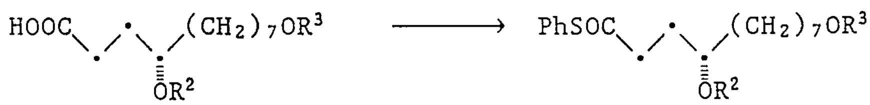

(4S)-19 (4S)-20

Ph bedeutet Phenyl.
Zu 2,54 g (6,13 mMol) (4S)-19 in 30 ml $CHCl_3$ bei 0°C werden während 5 min. 953 µl (6,74 mMol) 1-Chlor-N,N,2-trimethylpropenylamin getropft. Anschliessend wird 1 h bei 0°C nachgerührt, mit 658 µl (6,44 mMol) Thiophenol und 982 µl (12,26 mMol) Pyridin versetzt und weitere 30 min. bei 0°C gerührt. Zur Aufarbeitung wird das Reaktionsgemisch in Diethylether aufgenommen, je einmal mit $H_2O$, NaOH 1N und Sole gewaschen, mit $Na_2SO_4$ getrocknet und am Rotationsverdampfer eingeengt. Chromatographie des Rückstandes an Kieselgel (Petroleumbenzin 40-60°/Essigester = 9:1) liefert 2,69 g (87 %) (4S)-20. $[\alpha]_D^{25}$ = +16,6° (c = 0,865, $CHCl_3$).

Beispiel 31:

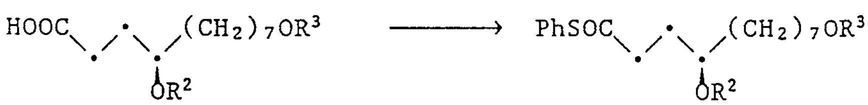

(4R)-19 (4R)-20

Analog zu Beispiel 30 werden ausgehend von 11,914 g (28,7 mMol) (4R)-19 12,24 g (85 %) (4R)-20 erhalten. $[\alpha]_D^{25}$ = -16,5°C (c = 0,985, $CHCl_3$).

Beispiel 32:

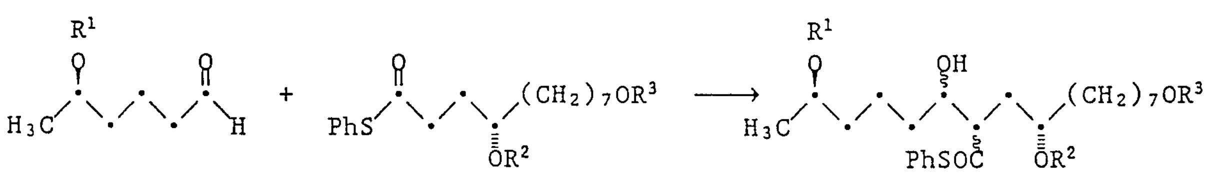

(5R)-7 (4S)-20 (8S,15R)-21

Bei -78°C wird eine Lösung von 2,69 g (5,3 mMol) Thioester (4S)-20 und 1,82 ml (10,62 mMol) Hünigbase in 40 ml Methylenchlorid während 5 min. mit 1,72 g (6,37 mMol) 9-BBNOTf versetzt. Anschliessend wird je 1 h bei

-78°C und 0°C gerührt. Nach erneutem Abkühlen auf -78°C werden 1,22 g (5,3 mMol) 5(R)-7 in 10 ml Methylenchlorid während 30 min. zugetropft. Anschliessend wird 30 min. bei -78°C und 1 h bei Raumtemperatur nachgerührt. Nach erneutem Abkühlen auf 0°C werden 10 ml Phosphatpuffer pH 7, 5 ml Methanol und 10 ml Perhydrol zugetropft und 1 h nachgerührt. Zur Aufarbeitung wird das Reaktionsgemisch in Diethylether aufgenommen und je 1x mit $H_2O$ und Sole gewaschen. Nach dem Trocknen mit $Na_2SO_4$ wird das Lösungsmittel abdestilliert und das so erhaltene Rohprodukt durch Chromatographie an Kieselgel (Petroleumbenzin 40-60°C/Essigester = 6:1) gereinigt. Dabei fallen 3,29 g (84 %) des Isomeren (8S,15R)-20 an.
NMR (300 MHz, $CDCl_3$): 7,45-7,23 (m, 10H), 6,82 (s br. 4H); 4,57 und 4,50 (AB, J = 12, ein $CH_2Ph$); 4,50 (s br. ein $CH_2Ph$); 3,90 (t, J = 7, 2H-C(1)); 3,90 (m, CH-O), 3,76 (m, CH-O); 3,75 (s, $OCH_3$), 3,62-3,43 (m, CH-O); 3,05 und 2,83 (2m, je ein CH); 2,80 und 2,30(2d, J = 4, je ein OH); 2,20-1,23 (übrige aliphatische Protonen); 1,12 (d, J = 7, $CH_3$); 0,88 (s br. $C(CH_3)_3$); 0,05 (s br. 2 $CH_3$).

Beispiel 33:

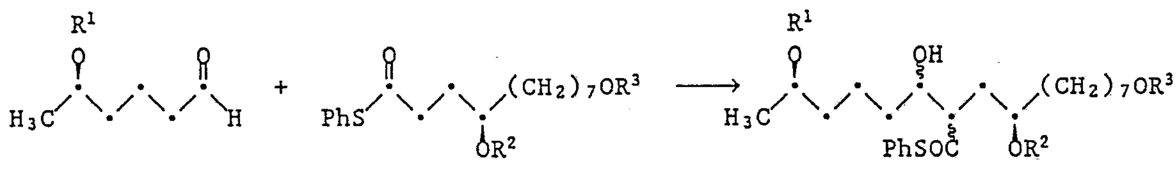

(5R)-7 (4R)-20 (8R,15R)-21

Analog zu Beispiel 32 werden aus 6,360 g (12,6 mMol) (4R)-20 und 2,893 g (12,6 mMol) (5R)-7 7,673 g (83 %) (8R,15R)-21 erhalten.
NMR (360 MHz, $CDCl_3$): 7,43-7,25 (m, 10H); 6,82 (s br. 4H); 4,58 und 4,49 (AB, J = 10, ein $CH_2Ph$); 4,53 und 4,50 (AB, J = 12, ein $CH_2Ph$); 3,82 (t, J = 7, 2H-C(1)); 3,82 (m, CH-O); 3,78 (m, CH-O); 3,76 (s, $OCH_3$); 3,57-3,47 (m, CH-O); 3,07 und 2,82 (2m, je ein CH); 2,22-1,25 (übrige aliphatische Protonen); 1,12 und 1,11 (2d, J = 7, je ein $CH_3$); 0,83 (s br. $C(CH_3)_3$); 0,05 (s br. 2 $CH_3$).

Beispiel 34:

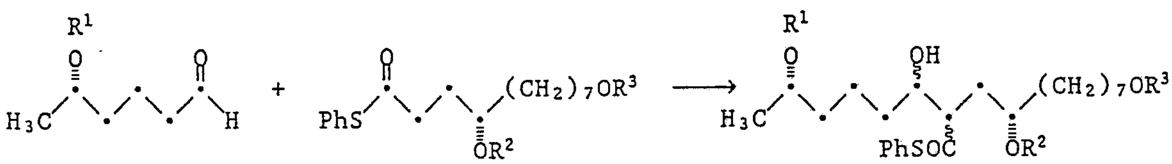

(5S)-7 (4S)-20 (8S,15S)-21

Analog zu Beispiel 32 werden aus 4,07 g (8,0 mMol) (4S)-20 und 1,851 g (8,0 mMol) (5S)-7 5,065 g (86 %) (8S,15S)-21 erhalten.
NMR (300 MHz, $CDCl_3$): 7,45-7,25 (m, 10H); 6,82 (s br. 4H); 4,57 und 4,50 (AB, J = 11, ein $CH_2Ph$); 4,52 (s br. ein $CH_2Ph$); 3,90 (t, J = 7, 2H-C(1)); 3,90 (m, CH-O); 3,78 (m, CH-O); 3,77 (s, $OCH_3$); 3,62-3,47 (m, CH-O); 3,07 und 2,82 (2m, je ein CH); 2,25-1,25 (übrige aliphatische Protonen); 1,13 und 1,12 (2d, J = 7, je ein $CH_3$); 0,87 (s br. $C(CH_3)_3$); 0,05 (s, 2 $CH_3$).

Beispiel 35:

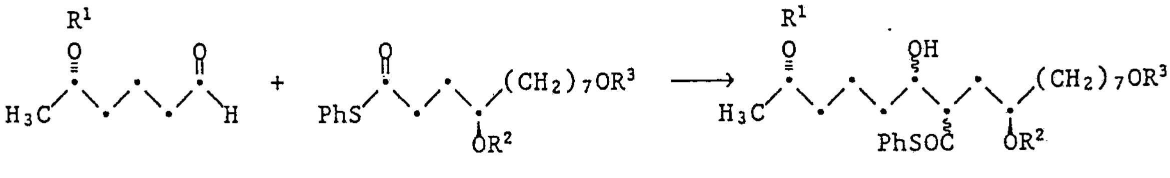

(5S)-7 (4R)-20 (8R,15S)-21

Analog zu Beispiel 32 wird aus 3,96 g (7,8 mMol) (4R)-20 und 1,801 g (7,8 mMol) (5S)-7 4,607 g (80 %) (8R,15S)-21 erhalten.
NMR (360 MHz, $CDCl_3$): 7,43-7,23 (m, 10H); 6,82 (s br. 4H); 4,57 und 4,45 (AB, J = 11, ein $CH_2Ph$); 4,52 und 4,45 (AB, J = 15, ein $CH_2Ph$); 3,89 (t, J = 7, 2H-C(1)); 3,89 (m, CH-O); 3,77 (m, CH-O); 3,76 (s, $OCH_3$); 3,62-3,47 (m, CH-O); 3,06 und 2,83 (2m, je ein CH), 2,80 und 2,31 (2d, J = 4, je ein OH); 2,22-1,25 (übrige aliphatische Protonen); 1,12 (d, J = 7, $CH_3$); 0,88 (s br. $C(CH_3)_3$); 0,05 (s br. 2 $CH_3$).

Beispiel 36:

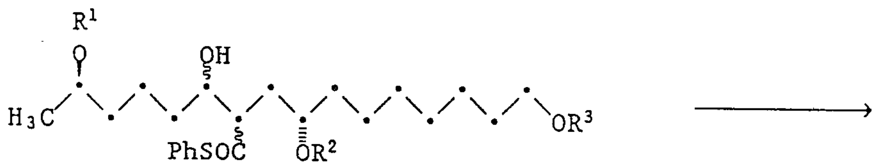

(8S,15R)-21

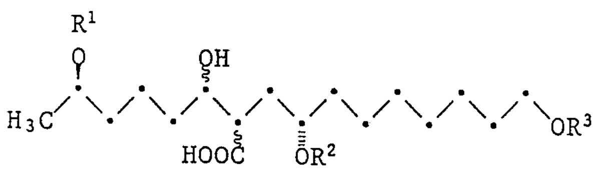

(8S,15R)-22

3,77 g (5,1 mMol) (8S,15R)-21 und 858 mg (20,5 mMol) LiOH in 60 ml Methanol und 20 ml $H_2O$ werden 17 h bei Raumtemperatur gerührt. Anschliessend wird das Reaktionsgemisch mit HCl 1N auf pH 1 gestellt, Methanol am Rotationsverdampfer abdestilliert und der Rückstand in Diethylether aufgenommen. Die Etherphase wird einmal mit Sole gewaschen, mit $Na_2SO_4$ getrocknet und am Rotationsverdampfer eingeengt. Der Rückstand wird mit 12,5 ml $H_2O$, 25 ml Dioxan und 1,72 g (43,1 mMol) NaOH versetzt und 90 min. am Rückfluss erhitzt. Anschliessend wird das Reaktionsgemisch eingeengt, der Rückstand mit $H_2O$ verdünnt und mit HCl 2N auf Ph 1 gestellt. Die wässrige Phase wird mit Diethylether dreimal extrahiert. Die vereinigten organischen Phasen werden mit $Na_2SO_4$ getrocknet und am Rotationsverdampfer eingeengt. Dabei werden 2,925 g (89 %) (8S,15R)-22 erhalten.
NMR (300 MHz, $CDCl_3$): 1,10 (d, J = 7, $CH_3$).

Beispiel 37:

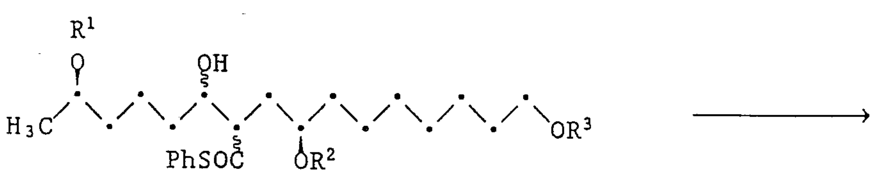

(8R,15R)-21

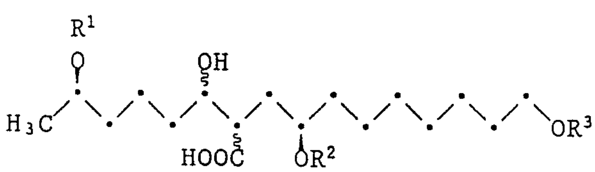

(8R,15R)-22

Analog zu Beispiel 36 wird ausgehend von 7,597 g (10,3 mMol) (8R,15R)-21 6,1 g (92 %) (8R,15R)-22 erhalten.

NMR (360 MHz, $CDCl_3$): 1,11 und 1,10 (2d, J = 7, je eine $CH_3$).

Beispiel 38:

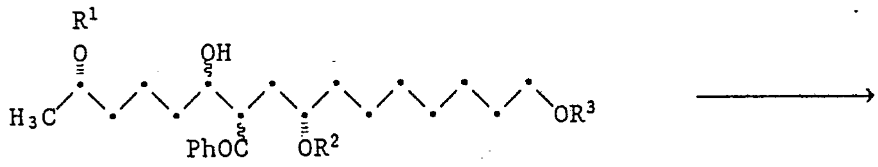

(8S,15S)-21

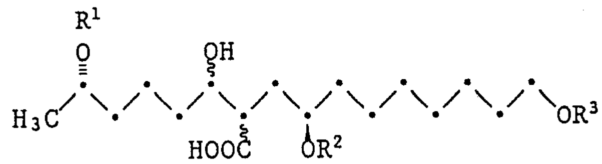

(8S,15S)-22

Analog zu Beispiel 36 werden ausgehend von 3,0 g (4,1 mMol) (8S,15S)-21 2,43 g (92 %) (8S,15S)-22 erhalten.
NMR (300 MHz, $CDCl_3$): 1,11 und 1,12 (2d, J = 7, je eine $CH_3$).

Beispiel 39:

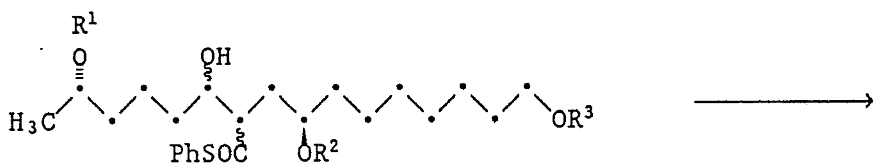

(8R,15S)-21

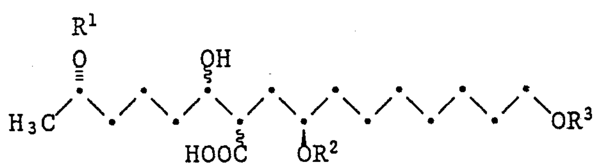

(8R,15S)-22

Analog zu Beispiel 36 werden ausgehend von 2,0 g (2,70 mMol) (8R,15S)-21 1,51 g (87 %) (8R,15S)-22 erhalten.
NMR (300 MHz, $CDCl_3$): 1,09 (d, J = 7, $CH_3$).

Beispiel 40:

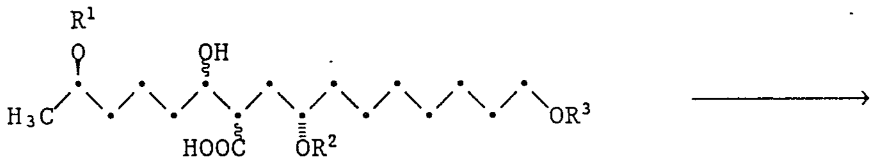

(8S,15R)-22

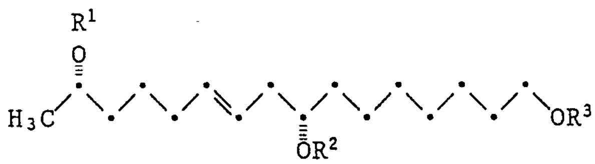

(8S,15R)-23

2,773 g (4,30 mMol) (8S,15R)-22 in 7,20 ml (25,8 mMol) Dineopentylacetal von DMF in 280 ml Xylol werden 30 min. am Rückfluss erhitzt. Das Reaktionsgemisch wird eingeengt und an Kieselgel (Petroleumbenzin 40-60°/Essigester - 9:1) chromatographiert. Dabei fallen 2,211 g (88 %) des 8S,15R-Isomeren 23 an.
$[\alpha]_D^{25}$ = -19,0° (0,99, $CHCl_3$);
NMR (300 MHz, $CDCl_3$): 1,09 (d, J = 7, $CH_3$).

Beispiel 41:

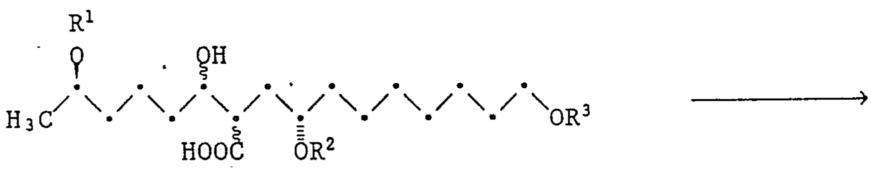

(8R,15R)-22

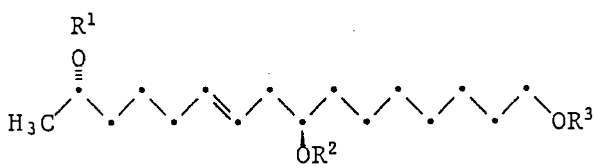

(8R,15R)-23

Analog zu Beispiel 40 werden ausgehend von 5,80 g (9 mMol) (8R,15R)-22 4,266 g (81 %) (8R,15R)-23 erhalten.
$[\alpha]_D^{25}$ = +5,6° (c = 1,419, $CHCL_3$).
NMR (300 MHz, $CDCl_3$): 1,11 (d, J = 7, $CH_3$).

Beispiel 42:

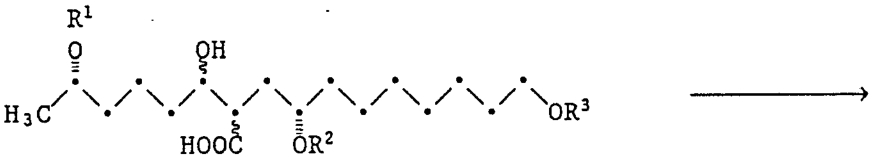

(8S,15S)-22

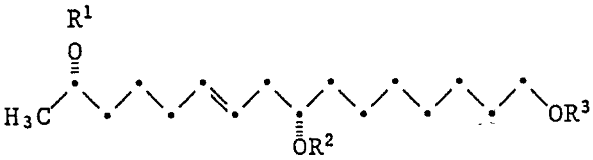

(8S,15S)-23

Analog zu Beispiel 40 werden ausgehend von 2,165 g (3,35 mMol) (8S,15S)-22 1,55 g (78 %) (8S,15S)-23 erhalten.
$[\alpha]_D^{25}$ = -4,8° (c = 1,525, $CHCl_3$);
NMR (300 MHz, $CDCl_3$): 1,12 (d, J = 7, $CH_3$).

Beispiel 43:

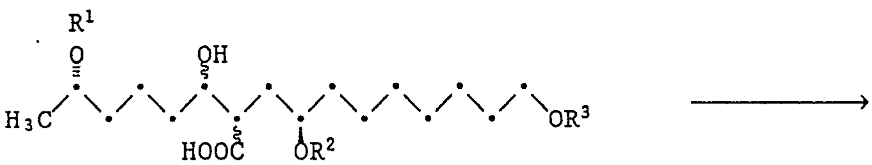

(8R,15S)-22

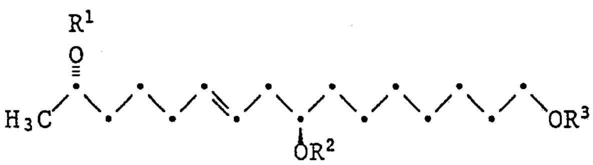

(8R,15S)-23

Analog zu Beispiel 40 werden ausgehend von 1,268 g (1,96 mMol) (8R,15S)-22 910 mg (80 %) (8R,15S)-23 erhalten.
$[\alpha]_D^{25}$ = +19,3° (c = 1,278, $CHCl_3$);
NMR (300 MHz, $CDCl_3$): 1,10 (d, J = 7, $CH_3$).

Beispiel 44:

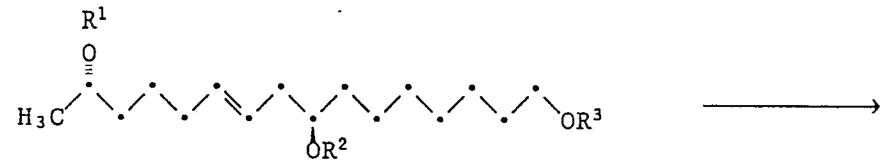

(8S,15R)-23

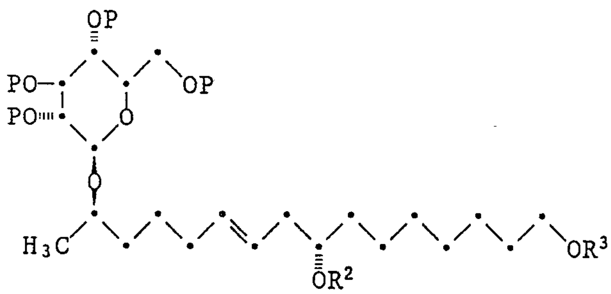

(8S,15R)-24

474 mg (0,813 mMol) (8S,15R)-23 und 421 mg (0,831 mMol) 2,3,4,6-Tetra-o-pivaloyl-α-D-fluorglucose in 1 ml Methylenchlorid werden bei Raumtemperatur während 10 min. mit 425 μ (3,415 mMol) Bortrifluoridetherat versetzt. Nach 50 min. wird mit Diethylether verdünnt, je 1 mal mit $H_2O$, gesättigter $NaHCO_3$ und Sole gewaschen, mit $Na_2SO_4$ getrocknet und eingeengt. Das Rohprodukt wird an Kieselgel (Petroleumbenzin 40-60° C/Essigester = 6:1) chromatographiert. Dabei fallen 557 mg (71 %) (8S,15R)-24 an.
$[\alpha]_D^{25}$ = -13,4° (c = 2,835 in $CHCl_3$);
NMR (300 MHz, $CDCl_3$): 1,08 (d, J = 7, $CH_3$).

Beispiel 45:

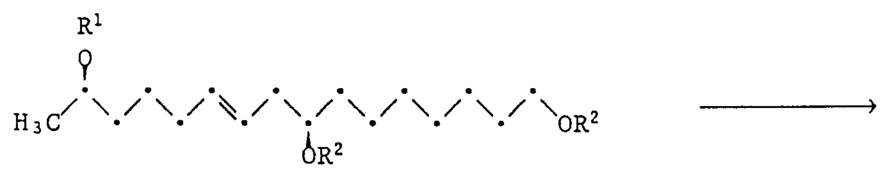

(8R,15R)-23

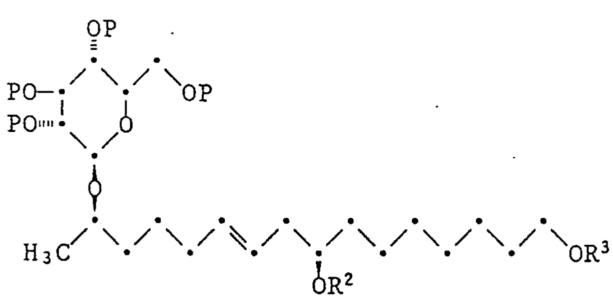

(8S,15R)-24

Analog zu Beispiel 44 werden ausgehend von 2,0 g (3,431 mMol) (8R,15R)-23 2,282 g (70 %) (8R,15R)-24 erhalten.
$[\alpha]_D^{25}$ = -2,6° (c = 0,930, $CHCl_3$);
NMR (360 MHz, $CDCl_3$): 1,08 (d, J = 7, $CH_3$).

Beispiel 46:

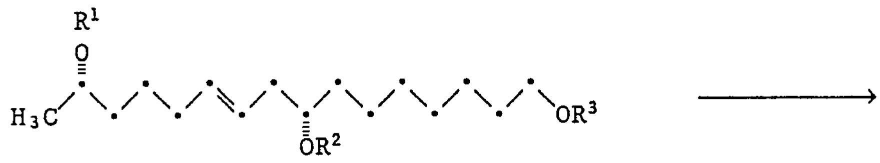

(8S,15S)-23

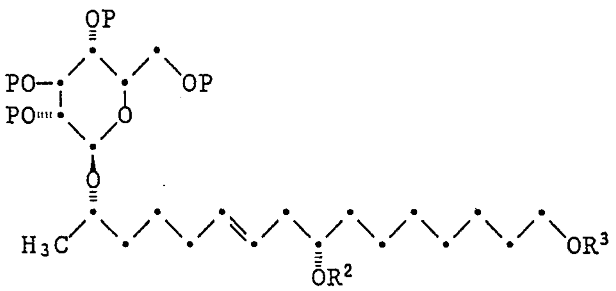

(8S,15S)-24

Analog Beispiel 44 werden ausgehend von 1,413 g (2,42 mMol) (8S,15S)-23 1,601 (69 %) (8S,15S)-24 erhalten.
$[\alpha]_D^{25} = -3{,}5°$ (c = 1,502, $CHCl_3$).
NMR (300 MHz, $CDCl_3$): 1,21 (d, J = 7, $CH_3$).

Beispiel 47:

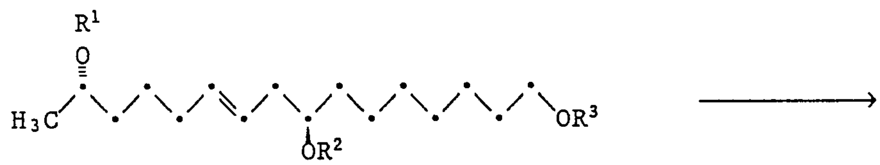

(8R,15S)-23

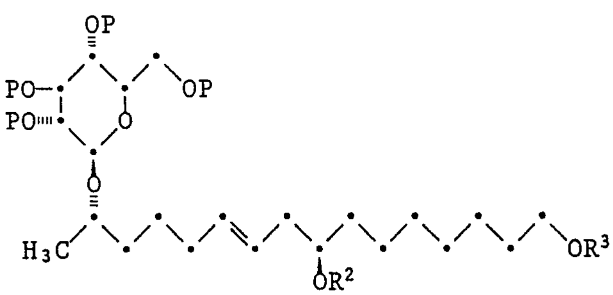

(8R,15S)-24

Analog Beispiel 44 werden ausgehend von 743 mg (1,274 mMol) (8R,15S)-23 831 mg (67 %) (8R,15S)-24 erhalten.
$[\alpha]_D^{25} = +7{,}0°$ (c = 1,085, $CHCl_3$);
NMR (300 MHz, $CDCl_3$): 1,20 (d, J = 7, $CH_3$).

Beispiel 48:

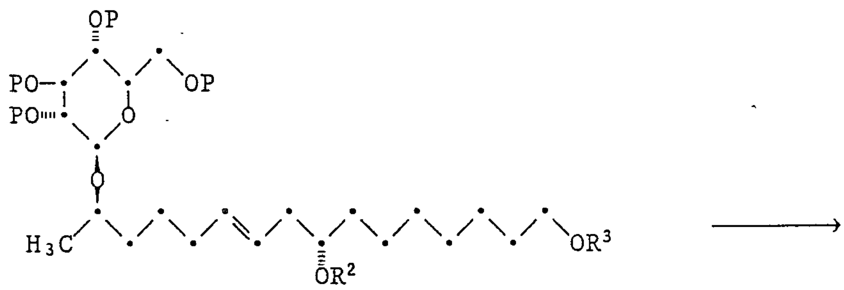

(8S,15R)-24

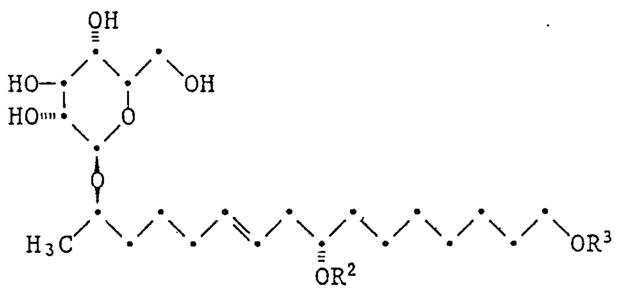

(8S,15R)-25

2,498 g (2,58 mMol) (8S,15R)-24 in 25 ml Methanol und einer katalytischen Menge Na werden im Bombenrohr bei 100°C 18 h gerührt. Anschliessend wird das Reaktionsgemisch eingeengt und an Kieselgel (Chloroform/Isopropanol = 5:1) chromatographiert. Dabei fallen 1,662 g (quantitativ) (8S,15R)-25 an. NMR (300 MHz, $CDCl_3$): 7,33 (m, 5H); 6,83 (s br. 4H); 5,46 (m, 2H); 4,56 und 4,48 (AB, J = 12, $CH_2Ph$); 4,35 (d, J = 7, H-C(1')); 3.90 (t, J = 7, 2H-C(1)); 3,77 (s, $OCH_3$); 3,92-3,72 (m, 3H); 3,57 (m, 2H); 3,46-3,25 (m, 3H); 2,30-1,10 (übrige aliphatische Protonen); 1,17 (d, J = 7, $CH_3$); $[\alpha]_D^{25}$ = -27,25° (c = 1,706, $CHCl_3$).

Beispiel 49:

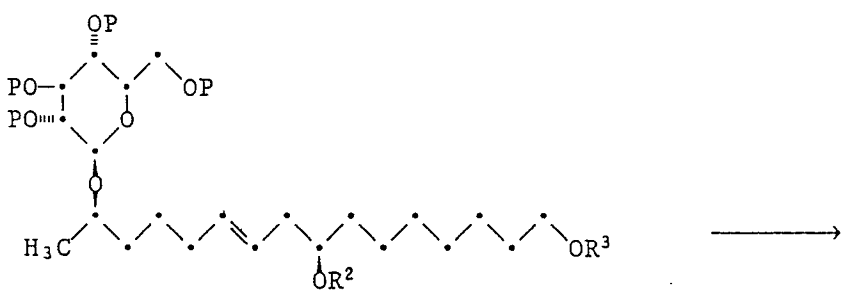

(8R,15R)-24

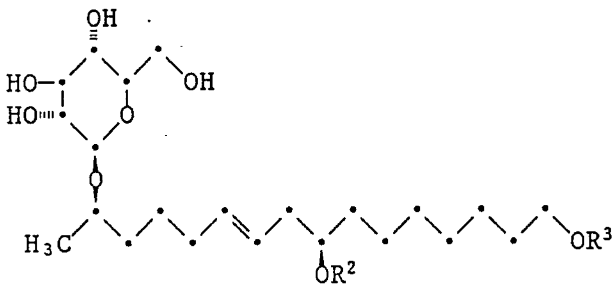

(8R,15R)-25

Analog Beispiel 48 werden ausgehend von 2,179 g (2,252 mMol) (8R,15R)-24 1,395 (96 %) (8R,15R)-25 erhalten.

$[\alpha]_D^{25}$ = -10,4° (c = 0,673, $CHCl_3$).

NMR (360 MHz, $CDCl_3$): 7,32 (m, 5H); 6,80 (s br. 4H); 5,43 (m, 2H); 4,53 und 4,46 (AB, J = 11, $CH_2Ph$); 4,32 (d, J = 7, H-C(1)); 3.87 (t, J = 7, 2H-C(1)); 3,83-3,70 (m, 3H); 3,74 (s, $OCH_3$); 3,57 (t, J = 7, 1H); 3,50 (t, J = 7, 1H); 3,42-3,21 (m, 3H); 2,23 (m, 2H); 2,0 (m, 2H); 1,72 (m, 2H); 1,68-1,10 (übrige aliphatische Protonen); 1,15 (d, J = 7, $CH_3$).

Beispiel 50:

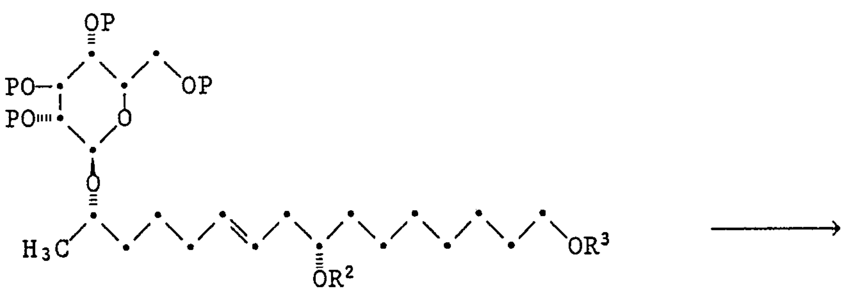

(8S,15S)-24

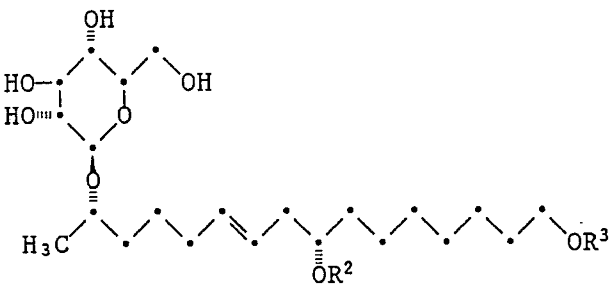

(8S,15S)-25

Analog Beispiel 48 werden ausgehend von 1,449 g (1,498 mMol) (8S,15S)-24 940 mg (quantitativ) (8S,15S)-25 erhalten.

$[\alpha]_D^{25}$ = -23,8° (c = 0,703, $CHCl_3$);

NMR (300 MHz, $CDCl_3$): 7,33 (m, 5H); 6,83 (s br. 4H); 5,45 (m, 2H); 4,55 und 4,47 (AB, J = 12, $CH_2Ph$); 4,34 (d, J = 7, H-C(1')); 3.88 (t, J = 7, 2H-C(1)); 3,91-3,68 (m, 3H); 3,76 (s, $OCH_3$); 3,58 (t, J = 8, 1H); 3,50 (t, J = 8, 1H); 3,45-3,25 (m, 3H); 2,25 (m, 2H); 2,01 (m, 2H); 1,73 (m, 2H); 1,80-1,11 (übrige aliphatische Protonen); 1,23 (d, J = 7, $CH_3$).

Beispiel 51:

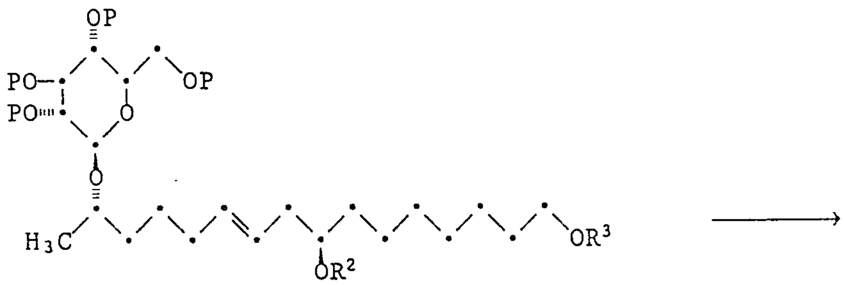

(8R,15S)-24

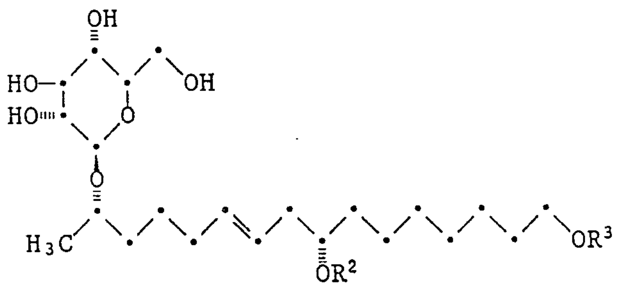

(8R,15S)-25

Analog Beispiel 48 werden ausgehend von 714 mg (0,738 mMol) (8R,15S)-24 453 mg (97 %) (8R,15S)-25 erhalten.

$[\alpha]_D^{25}$ = -10,6° (c = 0,974, $CHCl_3$).

NMR (300 MHz, $CDCl_3$): 7,33 (m, 5H); 6,83 (s br. 4H); 5,44 (m, 2H); 4,46 und 4,53 (AB, J = 11, $CH_2Ph$); 4,34 (d, J = 7, H-C(1)), 3,88 (t, J = 7, 2H-C(1)); 3,92-3,65 (m, 3H); 3,75 (s, $OCH_3$); 3,60 (t, J = 8, 1H); 3,50 (t, J = 8, 1H); 3,44-3,23 (m, 3H); 2,24 (m, 2H); 2,01 (m, 2H); 1,73 (m, 2H); 1,82-1,15 (übrige aliphatische Protonen); 1,22 (d, J = 7, $CH_3$).

Beispiel 52:

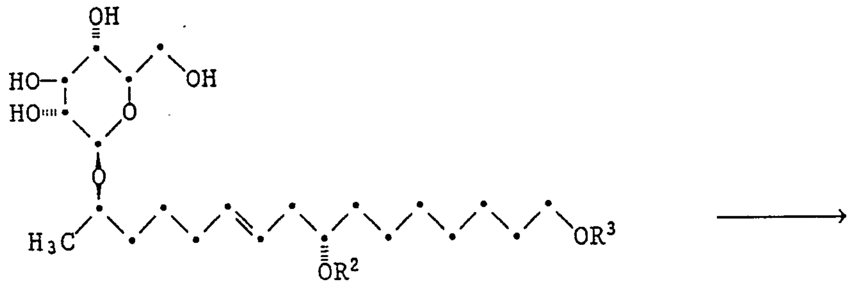

(8S,15R)-25

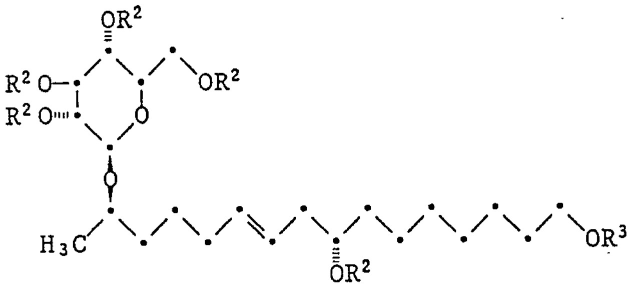

(8S,15R)-26

292 mg 55%ige NaH-Dispersion (6,683 mMol) werden unter Argon mit drei 3 ml Portionen Pentan gewaschen. Anschliessend werden 527 mg (0,835 mMol) (8S,15R)-26, 795 µl (6,683 mMol) Benzylbromid und 6 ml DMF zugegeben und das Reaktionsgemisch 30 min. bei Raumtemperatur gerührt. Zur Aufarbeitung wird mit 254 mg (3,34 mMol) Thioharnstoff versetzt, 1 h bei Raumtemperatur gerührt, mit Diethylether verdünnt und je einmal mit $H_2O$, $H_2O$:Sole = 1:1 und Sole gewaschen. Nach dem Trocknen der organischen Phase mit $Na_2SO_4$ wird das Lösungsmittel am Rotationsverdampfer entfernt. Das Rohprodukt wird an Kieselgel (Petroleumbenzin 40/60° C/Essigester = 6:1) chromatographiert. Dabei fallen 737 mg (89 %) (8S,15R)-27 an. NMR (300 MHz, $CDCl_3$): 7,38-7,13 (m, 25H); 6,82 (s br. 4H); 5,46 (m, 2H); 5,0-4,4 (5 AB-Systeme, $CH_2Ph$); 4,44 (d, J = 7, H-C(1′)); 3,88 (t, J = 7, 2H-C(1)); 3,76 (s, $OCH_3$); 3,77-3,30 (m, 8H); 2,23 (m, 2H); 3,04 (m, 2H); 2,74 (m, 2H); 2,74-1,23 (übrige alipatische Protonen); 1,20 (d, J = 7, $CH_3$).

Beispiel 53:

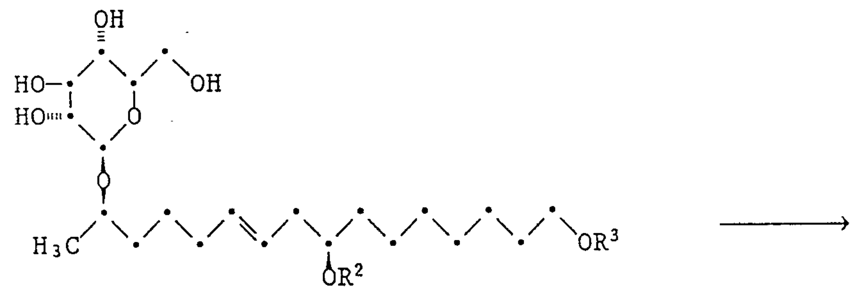

(8R,15R)-25

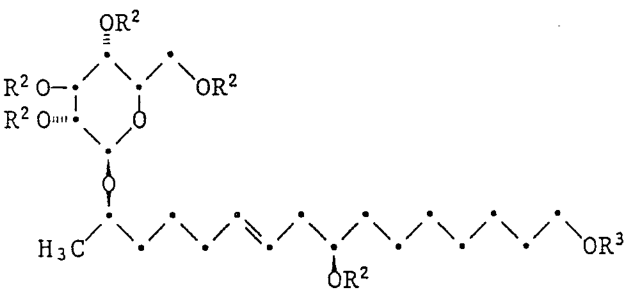

(8R,15R)-26

Analog Beispiel 52 werden ausgehend von 1,188 g (1,883 mMol) (8R,15R)-25 1,694 g (90 %) (8R,15R)-26 erhalten.
$[\alpha]^{25}_{D}$ = +7,8°, $[\alpha]^{25}_{365}$ = +29.3° (c = 0,714, $CHCl_3$).
NMR (300 MHz, $CDCl_3$): 1,20 (d, J = 7, $CH_3$).

Beispiel 54:

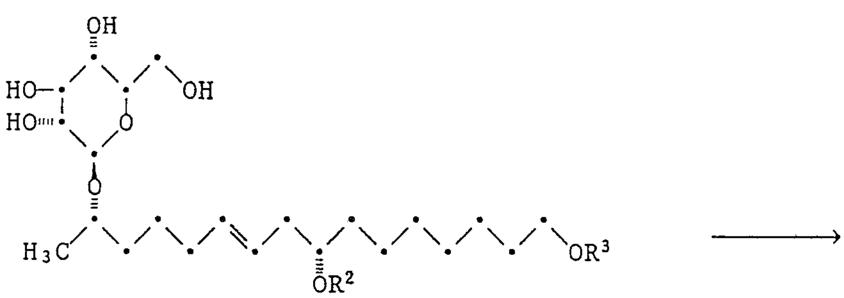

(8S,15S)-25

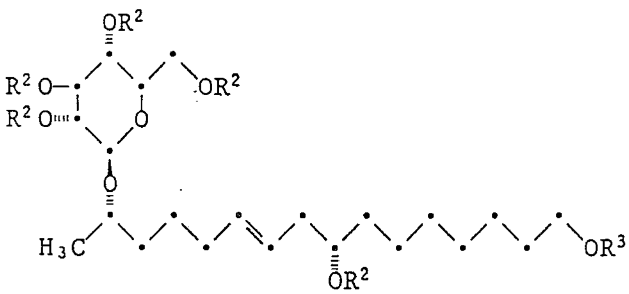

(8S,15S)-26

Analog Beispiel 52 werden ausgehend von 940 mg (8S,15S)-25 1,14 (77 %) (8S,15S)-26 erhalten.
$[\alpha]_D^{25} = +0,6°$, $[\alpha]$ $= +3°$ (c = 1,265, $CHCl_3$).
NMR (300 MHz, $CDCl_3$): 1,30 (d, J = 7, $CH_3$).

Beispiel 55:

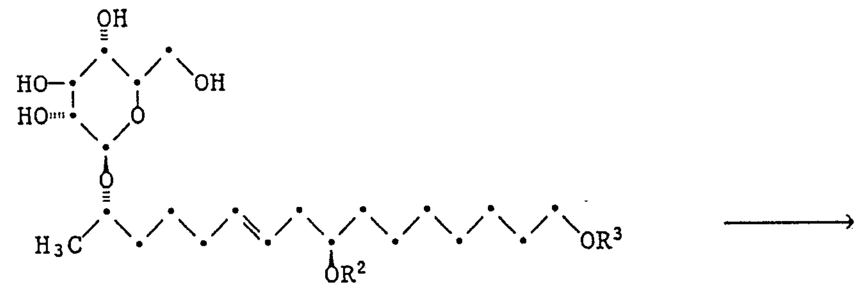

(8R,15S)-25

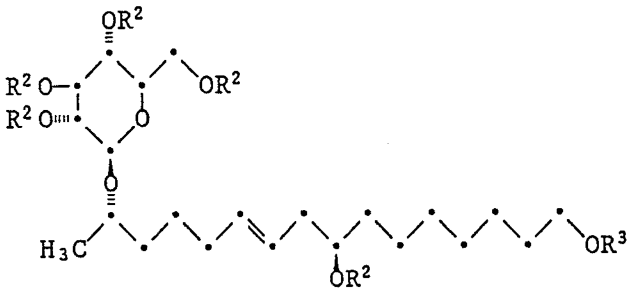

(8R,15S)-26

Analog Beispiel 52 werden ausgehend von 319 mg (0,506 mMol) (8R,15S)-25 468 mg (93 %) (8R,15S)-26 erhalten.
$[\alpha]_D^{25} = +11,7°$, $[\alpha]_{365}^{25} = +38,4°$ (c = 1,950, $CHCl_3$);

Analyse ($C_{64}H_{78}O_9$; 991,32):

| | | | |
|---|---|---|---|
| Berechnet: | C 77,54 % | H 7,93 % | O 14,53 % |
| Gefunden: | C 77,45 % | H 7,91 % | O 14,27 % |

NMR (300 MHz, $CDCl_3$): 1,30 (d, J = 7, $CH_3$).
Beispiel 56:

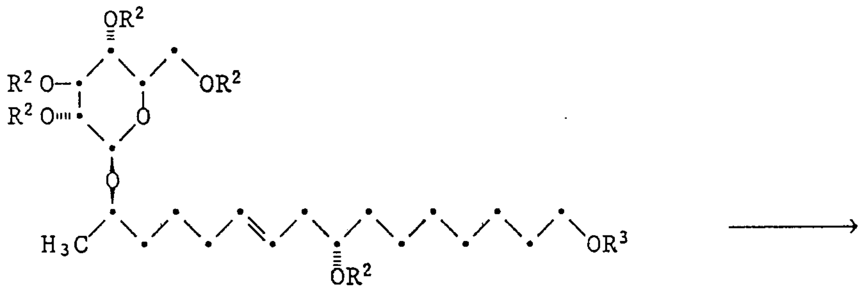

(8S,15R)-26

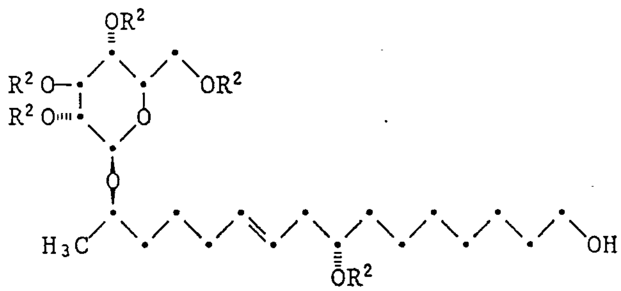

(8S,15R)-27

597 mg (0,602 mMol) (8S,15R)-26 und 1,15 g (2,108 mMol) Cerammoniumnitrat in 12 ml Acetonitril und 3 ml $H_2O$ werden bei 0° C 40 min. gerührt. Zur Aufarbeitung wird mit Diethylether verdünnt und je einmal mit $H_2O$, gesättigter $NaHCO_3$ und Sole gewaschen. Nach dem Trocknen der organischen Phase mit $Na_2SO_4$ wird das Lösungsmittel am Rotationsverdampfer entfernt. Der Rückstand wird an Kieselgel (Petroleumbenzin 40-60° C/Essigester = 3:1) chromatographiert. Dabei fallen 568 mg (78 %) (8S,15R)-27 an.
NMR (300 MHz, $CDCl_3$): 1,18 (d, J = 7, $CH_3$).

Beispiel 57:

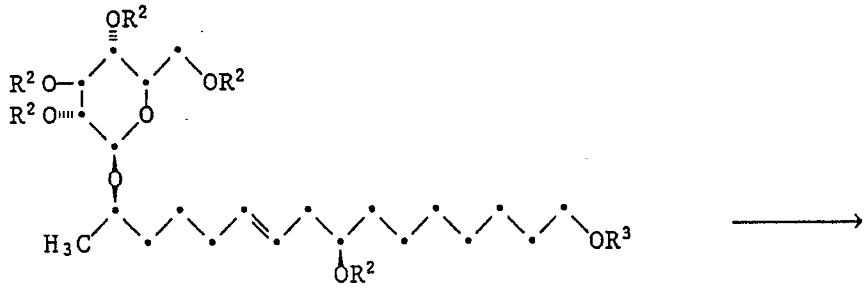

(8R,15R)-26

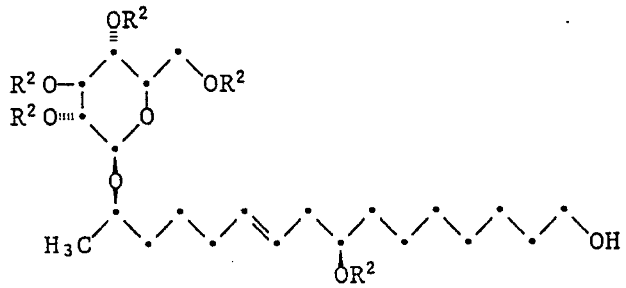

(8R,15R)-27

Analog Beispiel 56 werden ausgehend von 1,535 g (1,548 mMol) (8R,15R)-26 995 mg (73 %) (8R,15R)-27 erhalten.

$[\alpha]_D^{25} = +9{,}5°$, $[\alpha]_{365}^{20} = +23{,}6°$ (c = 0,704, $CHCl_3$);

NMR (300 MHz, $CDCl_3$): 1,20 (d, J = 7, $CH_3$).

Beispiel 58:

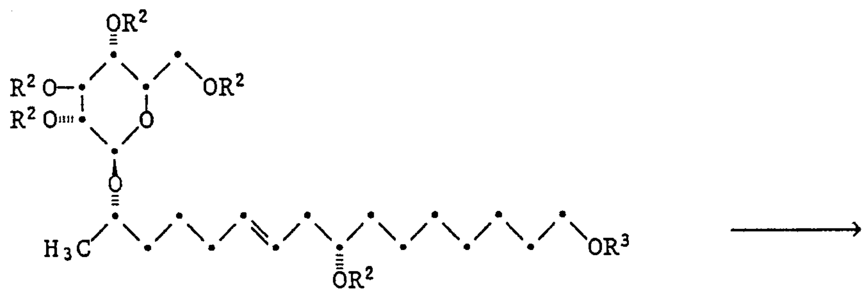

(8S,15S)-26

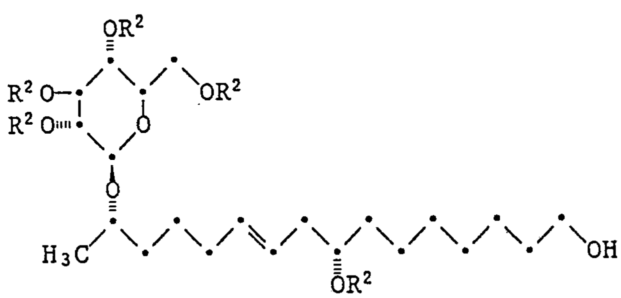

(8S,15S)-27

Analog Beispiel 56 werden ausgehend von 700 mg (0,706 mMol) (8R,15S)-26 460 mg (76 %) (8S,15S)-27 erhalten.
$[\alpha]_D^{20} = +0,0°$ (c = 0,816, $CHCl_3$);
NMR (300 MHz, $CDCl_3$): 1,30 (d, J = 7, $CH_3$).

Beispiel 59:

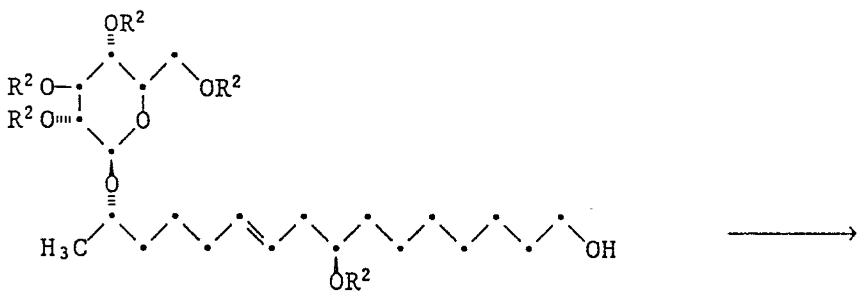

(8R,15S)-26

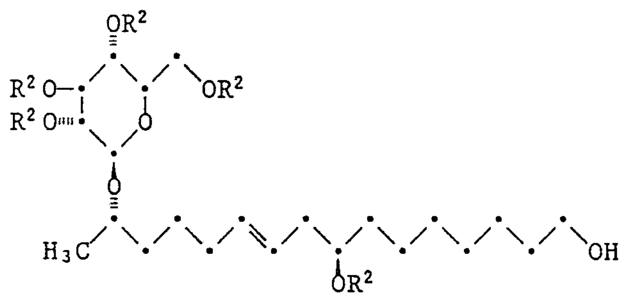

(8R,15S)-27

Analog Beispiel 56 werden ausgehend von 335 mg (0,337 mMol) (8R,15S)-26 249 mg (83 %) (8R,15S)-27 erhalten. $[\alpha]_D^{20} = +13,2°$ (c = 0,783, $CHCl_3$);
NMR (300 MHz, $CDCl_3$): 1,29 (d, J = 7, $CH_3$).

Beispiel 60:

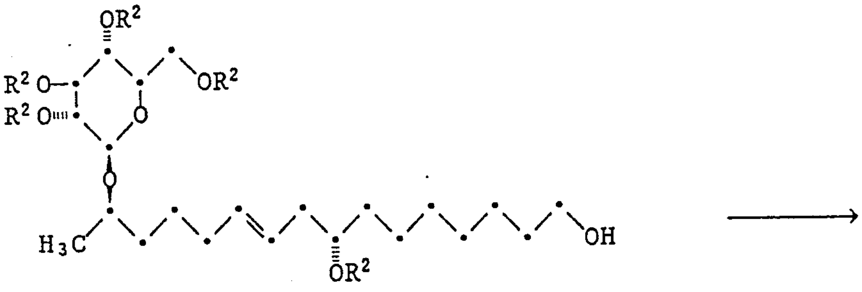

(8S,15R)-27

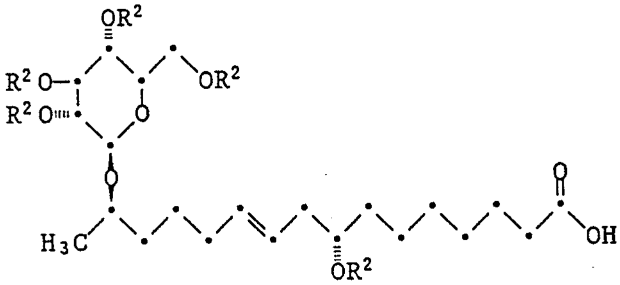

(8S,15R)-28

51 mg (0,0576 mMol) 8S,15R)-27 in 2 ml Aceton werden bei 0° C mit 51 µl einer 4N-Jones-Lösung versetzt und 30 min. bei 0° C gerührt. Anschliessend wird mit Diethylether verdünnt, an Watte filtriert und je einmal mit $H_2O$ und Sole gewaschen. Nach dem Trocknen mit $Na_2SO_4$ wird das Lösungsmittel am Rotationsverdampfer verdampft. Das Rohprodukt wird an Kieselgel (Petroleumbenzin 40-60° C/Essigester = 3:2) chromatographiert. Dabei werden 47 mg (90 %) der Carbonsäure (8S,15R)-28 erhalten.
NMR (300 MHz, $CDCl_3$): 7,39-7,12 (m, 25H); 5,45 (m, 2H); 5,0-4,4 (5 AB-Systeme, $CH_2Ph$); 4,45 (d, J = 7, H-C(1)); 3,88 (m, 1H); 3,5-3,8 (m, 4H), 3,5-3,3 (m, 3H); 2,31 (t, J = 7, 2H-C(2)); 2,23 (m, 2H); 2,04 (m, 2H); 1,73-1,16 (übrige aliphatische Protonen); 1,20 (d, J = 7, $CH_3$).

Beispiel 61:

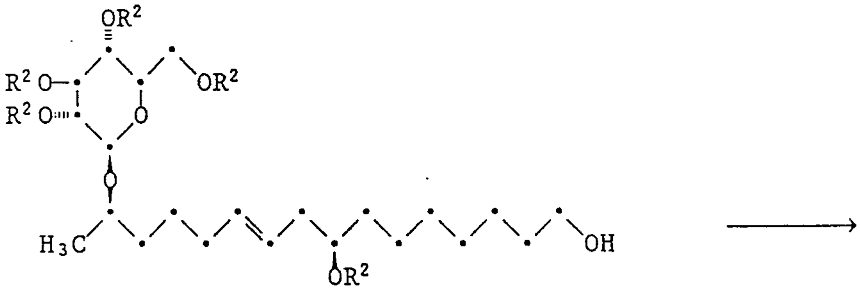

(8R,15R)-27

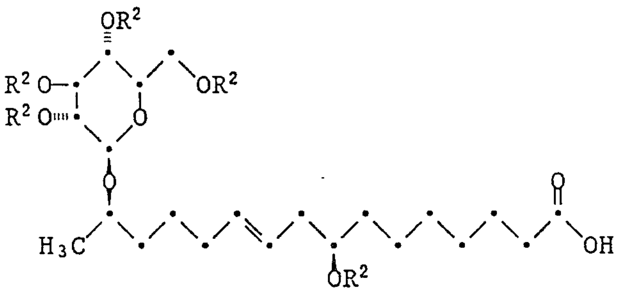

(8R,15R)-28

Analog Beispiel 60 werden ausgehend von 891 mg(1,006 mMol) (8R,15R)-27 646 mg (71 %) (8R,15R)-28 erhalten.
$[\alpha]_D^{20} = +9{,}3°$, $[\alpha]_{365}^{25} = +33{,}7°$ (c = 1,209, $CHCl_3$);
NMR (300 MHz, $CDCl_3$): 7,39-7,11 (m, 25H); 5,45 (m, 2H); 5,0-4,4 (5 AB-Systeme, $CH_2Ph$); 4,44 (d, J = 7, H-C(1')); 3,87 (m, 1H), 3,77-3,49 (m, 4H); 3,49-3,29 (m, 3H); 2,32 (t, J = 7, 2H-C(2)); 2,24 (m, 2H); 2,03 (m, 2H); 1,75-1,14 (übrige aliphatische Protonen); 1,19 (d, J = 7, $CH_3$).

Beispiel 62:

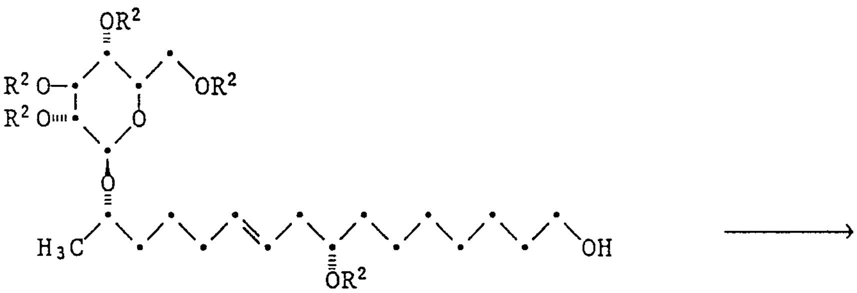

(8S,15S)-27

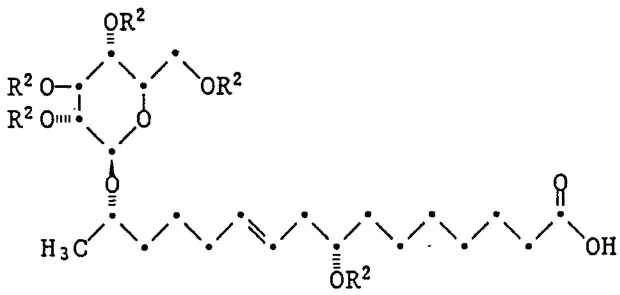

(8S,15S)-28

Analog Beispiel 60 werden ausgehend von 387 mg (0,437 mMol) (8S,15S)-27 329 mg (84 %) (8S,15S)-28 erhalten.

$[\alpha]_D^{20} = +0{,}6°$, $[\alpha]_{365}^{25} = +3{,}5°$ (c = 0,869, $CHCl_3$);

NMR (300 MHz, $CDCl_3$): 7,37-7,12 (m, 25H); 5,39 (m, 2H); 5,0-4,41 (5 AB-Systeme, $CH_2Ph$); 4,45 (d, J = 7, H-C(1)); 3,86-3,29 (m, 8H); 2,32 (t, J = 7, 2H-C(2)); 2,22 (m, 2H); 1,97 (m, 2H); 1,73-1,18 (übrige aliphatische Protonen); 1,29 (d, J = 7, $CH_3$).

Beispiel 63:

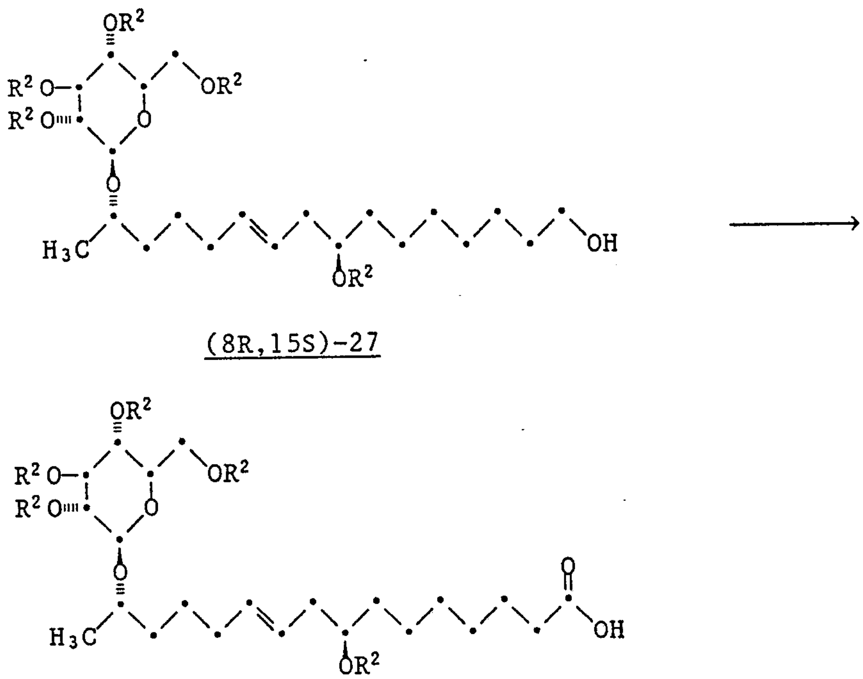

(8R,15S)-27

(8R,15S)-28

Analog Beispiel 60 werden ausgehend von 175 mg (0,197 mMol) (8R,15S)-27 154 mg (87 %) (8R,15S)-28 erhalten.

Beispiel 64:

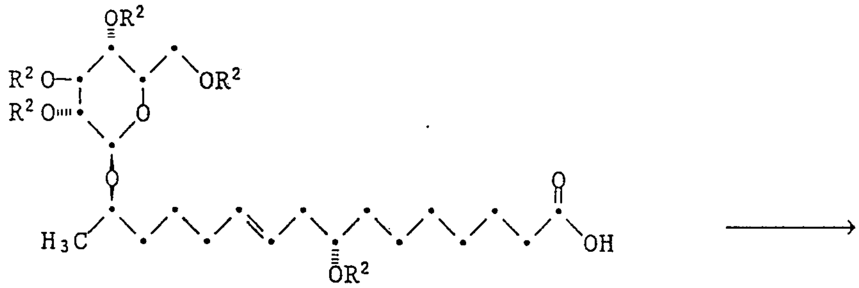

(8S,15R)-28

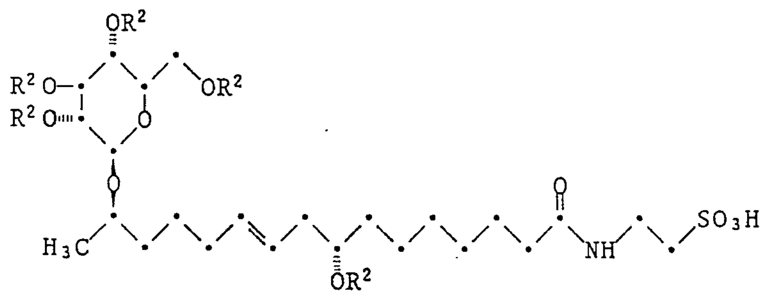

(8S,15R)-29

Ein Gemisch von 219 mg (0,243 mMol) (8S,15R)-28, 34 mg (0,292 mMol) Hydroxysuccinimid und 66 mg (0,316 mMol) Dicyclohexylcarbodiimid in 4 ml Dimethoxyethan wird bei Raumtemperatur 14 h gerührt. Anschliessend wird das Reaktionsgemisch filtriert, das Filtergut mit 10 ml Dimethoxyethan nachgespült und das Filtrat eingeengt. Der Rückstand wird in 1,5 ml Methanol gelöst und bei Raumtemperatur mit 30 mg (0,243 mMol) Taurin in 255 µl (0,255 mMol) NaOH 1N und 3 ml Methanol versetzt und 2 h bei Raumtemperatur gerührt. Dann wird das Reaktionsgemisch eingeengt und an Kieselgel (Chloroform/Methanol = 4:1) chromatographiert. Dabei fallen 241 mg (96 %) (8S,15R)-29 an.
NMR (300 MHz, $CDCl_3$): 1,17 (d, J = 7, $CH_3$).

Beispiel 65:

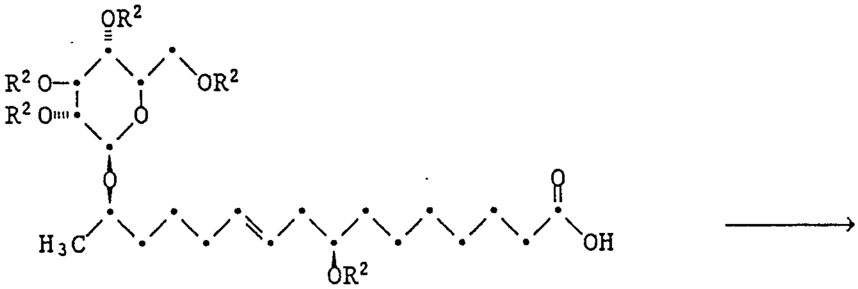

(8R,15R)-28

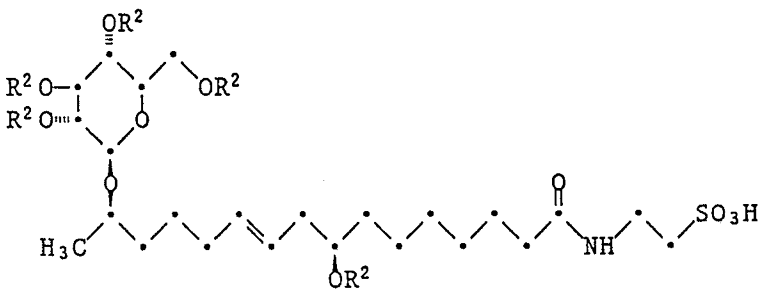

(8R,15R)-29

Analog Beispiel 64 werden ausgehend von 554 mg (0,616 mMol) (8R,15R)-28 532 mg (84 %) (8R,15R)-28 erhalten.
NMR (300 MHz, $CDCl_3$): 1,17 (d, J = 7, $CH_3$).

Beispiel 66:

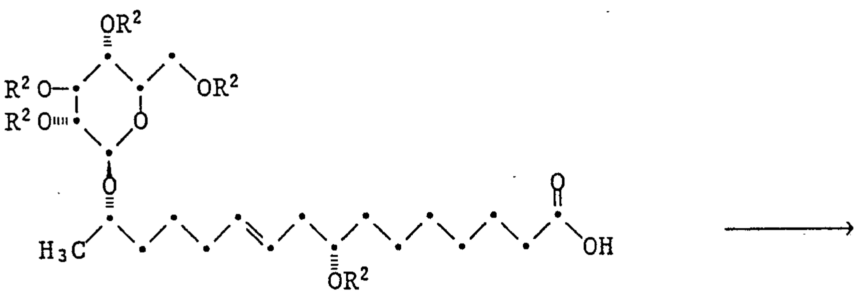

(8S,15S)-28

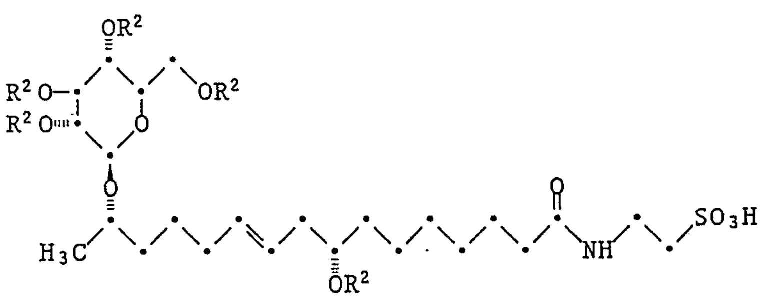

(8S,15S)-29

Analog Beispiel 64 werden ausgehend von 275 mg (0,306 mMol) (8S,15S)-28 310 mg (99 %) (8S,15S)-29 erhalten.
NMR (300 MHz, $CDCl_3$): 1,25 (d, J = 7, $CH_3$).

Beispiel 67:

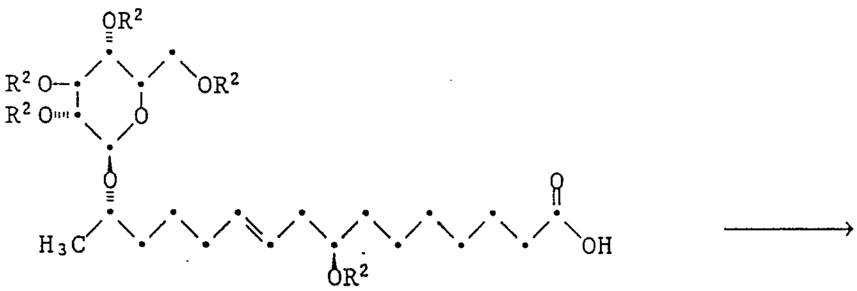

(8R,15S)-28

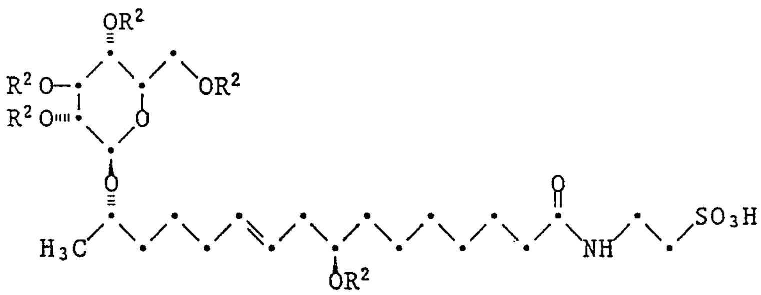

(8R,15S)-29

Analog Beispiel 64 werden ausgehend von 117 mg (0,130 mMol) 8R,15S)-28 77 mg (58 %) (8R,15S)-29 erhalten.
NMR (300 MHz, $CDCl_3$): 1,26 (d, J = 7, $CH_3$).

Beispiel 68:

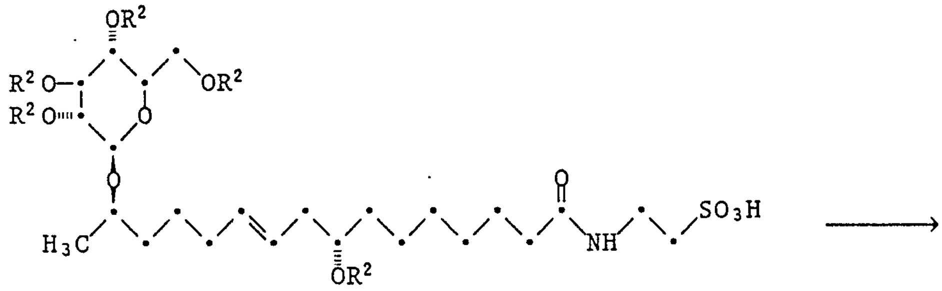

(8S,15R)-29

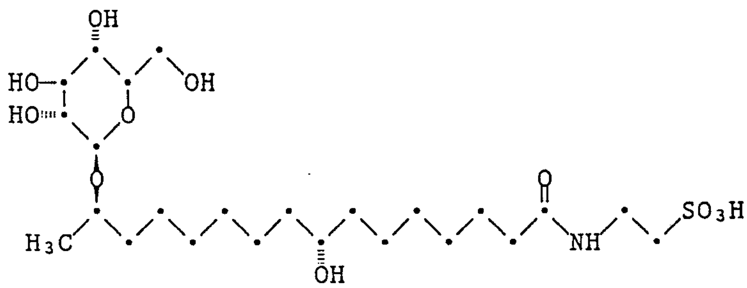

(8R,15R)-30

80 mg (0,0795 mMol) (8S,15R)-29 und 80 mg 10 % Pd/C in 2 ml Methanol werden 19 h bei Normaldruck hydriert. Anschliessend wird das Reaktionsgemisch an Celite filtriert und eingeengt. Der Rückstand wird an Kieselgel (OPTI-UP $C_{12}$, Antec AG) (Acetonitril/$H_2O$ = 9:1) chromatographiert. Dabei wurden 41 mg (93 %) (8R,15R)-30 erhalten.

$[\alpha]_D^{25}$ = -19,3° (c = 0,280, Methanol).

NMR (400 MHz, $CD_3OD$): 4,31 (d, J = 8, H-C(1')); 3,85 (m, H-C(15)); 3,84 (dd, $J_1$ = 12, $J_2$ = 2, H-C(6')); 3,66 (dd, $J_1$ = 12, $J_2$ = 6, H-C(6')); 3,58 (t, J = 7, S-$CH_2$); 3,50 (m, H-C(8)); 3,33 (t, J = 9, H-C(3')); 3,28 (t, J = 9, H-C(4')); 3,23 (ddd, $J_1$ = 9, $J_2$ = 6, $J_3$ = 2, H-C(5')); 3,13 (dd, $J_1$ = 9, $J_2$ = 8, H-C(2')); 2,95 (t, J = 7, N-$CH_2$); 2,18 (t, J = 7, 2H-C(2)); 1,65-1,25 (übrige aliphatische Protonen); 1,16 (d, J = 6, $CH_3$).

Beispiel 69:

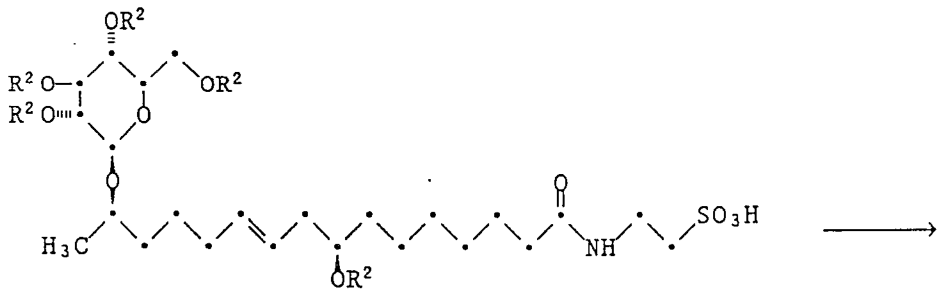

(8R,15R)-29

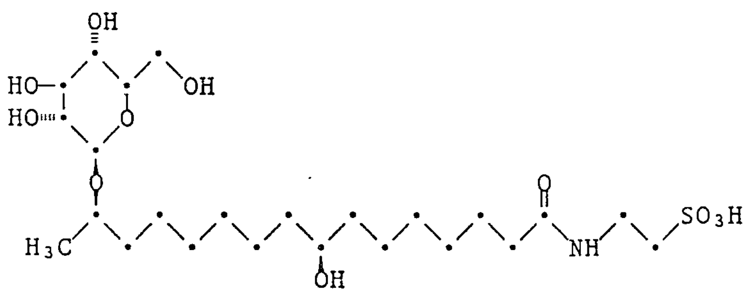

(8S,15R)-30

Analog Beispiel 68 werden ausgehend von 131 mg (0,1302 mMol) 8R,15R)-29 60 mg (82 %) (8S,15R)-30 erhalten.

$[\alpha]_D^{25}$ = -17,9° (c = 0,235, Methanol).

NMR (400 MHz, $CD_3OD$): 4,31 (d, J = 8, H-C(1′)); 3,85 (m, H-C(15)); 3,84 (dd, $J_1$ = 12, $J_2$ = 2, H-C(6′)); 3,66 (dd, $J_1$ = 12, $J_2$ = 6, H-C(6′)); 3,58 (t, J = 7, S-$CH_2$); 3,50 (m, H-C(8)); 3,33 (t, J = 9, H-C(3′)); 3,28 (t, J = 9, H-C(4′)); 3,23 (ddd, $J_1$ = 9, $J_2$ = 6, $J_3$ = 2, H-C(5′)); 3,13 (dd, $J_1$ = 9, $J_2$ = 8, H-C(2′)); 2,95 (t, J = 7, N-$CH_2$); 2,18 (t, J = 7, 2H-C(2)); 1,65-1,25 (übrige aliphatisiche Protonen); 1,16 (d, J = 6, $CH_3$).

Beispiel 70:

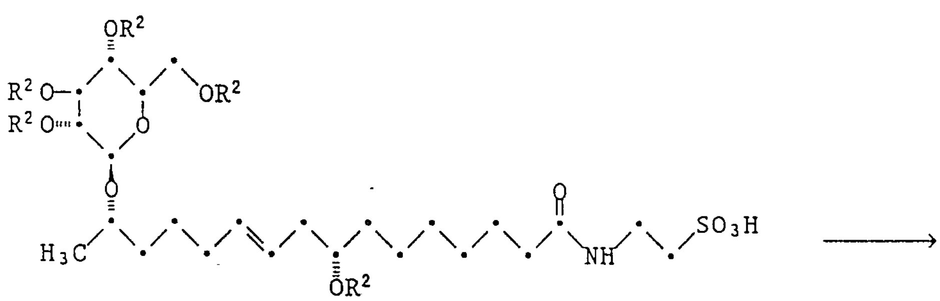

(8S,15S)-29

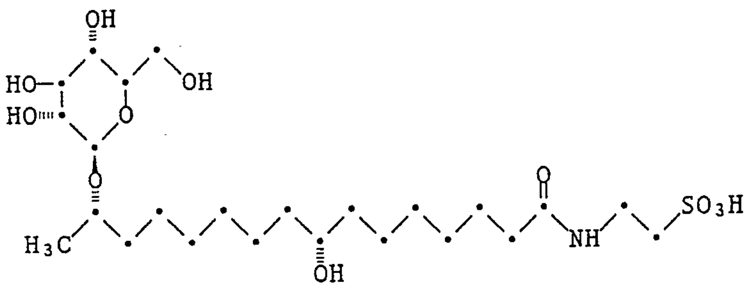

(8R,15S)-30

Analog Beispiel 68 werden ausgehend von 53 mg (0,0527 mMol) (8S,15S)-29 16 mg (55 %) (8R,15S)-30 erhalten.
$[\alpha]_D^{25}$ = -16,9° (c = 0,455, Methanol).
NMR (400 MHz, $CD_3OD$): 4,32 (d, J = 8, H-C(1')); 3,84 (dd, $J_1$ = 12, $J_2$ = 2, H-C(6')); 3,81 (m, H-C(15)); 3,65 (dd, $J_1$ = 12, $J_2$ = 5, H-C(6')); 3,58 (t, J = 7, S-$CH_2$); 3,49 (m, H-C(8)); 3,34 (t, J = 9, H-C(3')); 3,27 (t, J = 9, H-C(4')); 3,25 (ddd, $J_1$ = 9, $J_2$ = 6, $J_3$ = 2, H-C(5')); 3,13 (dd, $J_1$ = 9, $J_2$ = 8, H-C(2')); 2,95 (t, J = 7, N-$CH_2$); 2,17 (t, J = 7, 2H-C(2)); 1,65-1,25 (übrige aliphatische Protonen); 1,22 (d, J = 6, $CH_3$).

Beispiel 71:

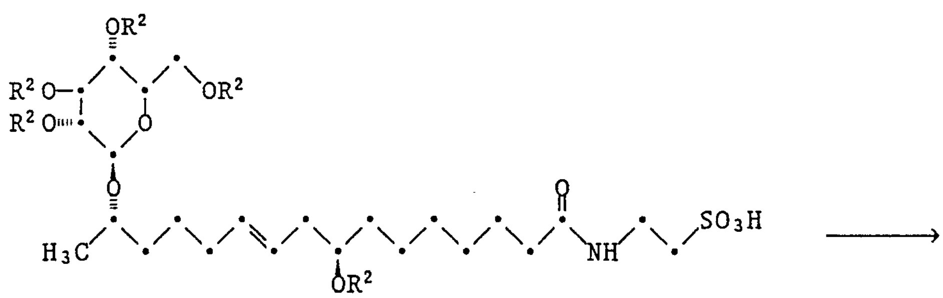

(8R,15S)-29

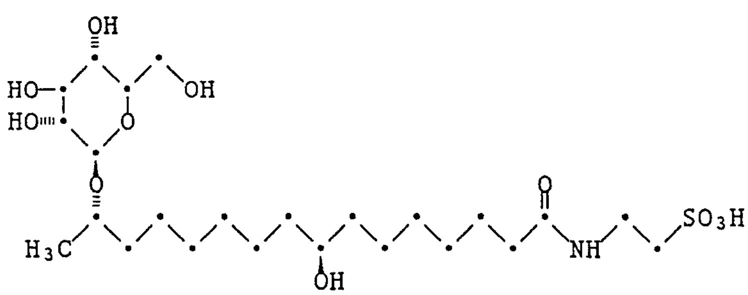

(8S,15S)-30

Analog Beispiel 68 werden ausgehend von 73 mg (0,0725 mMol) (8R,15S)-29 23 mg (58 %) (8S,15S)-30 erhalten.
$[\alpha]_D^{25}$ = -15,8° (c = 0,190, Methanol).
NMR (400 MHz, $CD_3OD$): 4,32 (d, J = 8, H-C(1)); 3,84 (dd, $J_1$ = 12, $J_2$ = 2, H-C(6')); 3,81 (m, H-C(15)); 3,65 (dd, $J_1$ = 12, $J_2$ = 5, H-C(6')); 3,58 (t, J = 7, S-$CH_2$); 3,49 (m, H-C(8)); 3,34 (t, J = 9, H-C(3')); 3,27 (t, J = 9, H-C(4')); 3,25 (ddd, $J_1$ = 9, $J_2$ = 6, $J_3$ = 2, H-C(5')); 3,13 (dd, $J_1$ = 9, $J_2$ = 8, H-C(2')); 2,95 (t, J = 7, N-$CH_2$); 2,17 (t, J = 7, 2H-C(2)); 1,65-1,25 (übrige aliphatische Protonen); 1,22 (d, J = 6, $CH_3$).

C. Elektrophysiologische Prüfung

Die Prüfung auf biologische Aktivität der 4 optischen Isomeren erfolgt mittels elektrophysiologischen Ableitungen von tarsalen Kontakt-Chemorezeptoren, wobei nach der von E. Städler beschriebenen Methode

vorgegangen wird (E. Städler: Contract Chemoreception; Chemical Ecology of Insects, 1984, p. 3-35). Die zu messenden Proben bestehen aus 10 µl Lösung aufgelöst in 1 ml 10 mM Natriumchlorid-Lösung. 2 µl dieser verdünnten Lösung werden in eine Glas-Kapillarelektrode aufgesogen und zur Stimulierung 4 bis 8 Rezeptoren des Prothorax tarsus einer weiblichen Kirschfliege verwendet. Die 4 optischen Isomeren zeigen alle eine biologische Aktivität.

**Patentansprüche**

1. Verbindung der Formel I

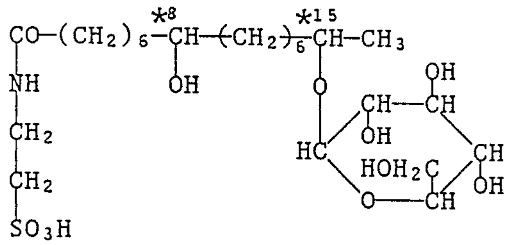

(I)

wobei * die Form ihrer optischen Isomeren (8R,15R), (8R,15S), (8S,15R) und (8S,15S) bedeutet, sowie ihre Alkali- und Erdalkalimetallsalze.

2. Verbindung gemäss Anspruch 1, dadurch gekennzeichnet, dass es sich bei den Salzen um ein Natrium-, Kalium- oder Magnesiumsalz handelt.

3. Verbindung gemäss Anspruch 1, dadurch gekennzeichnet, dass das Isomer 8R,15R-Konfiguration aufweist.

4. Verbindung gemäss Anspruch 1, dadurch gekennzeichnet, dass das Isomer 8S,15R-Konfiguration aufweist.

5. Verfahren zur Herstellung von Verbindungen der Formel I gemäss Anspruch 1, dadurch gekennzeichnet, dass man

a) eine Verbindung der Formel II

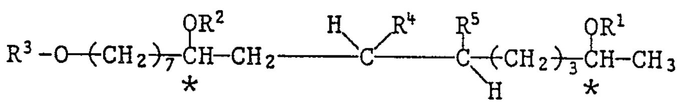

(II),

worin * die optischen Isomeren (R,R), (R,S), (S,R) und (S,S) bedeutet, $R^4$ und $R_5$ je für H oder beide zusammen für eine Bindung stehen und $R^1$, $R^2$ und $R^3$ unabhängig voneinander je eine Schutzgruppe bedeuten, mit in den 2,3,4,6-Stellungen geschützte OH-Gruppen enthaltender α-D-Fluorglucose in Gegenwart von $BF_3$ zu einer Verbindung der Formel III umsetzt,

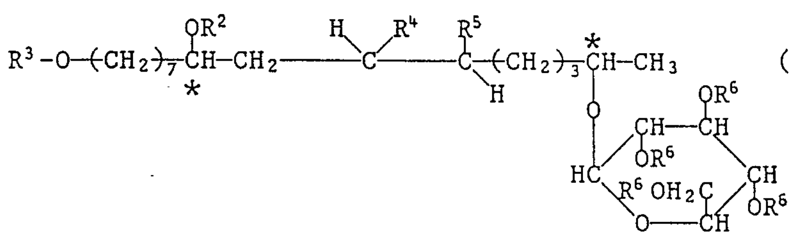

(III),

wobei $R^6$ eine Schutzgruppe bedeutet;

b) danach die Schutzgruppe $R^3$ entfernt und die erhaltene $-CH_2OH$-Gruppe zur Herstellung einer Verbindung der Formel IV

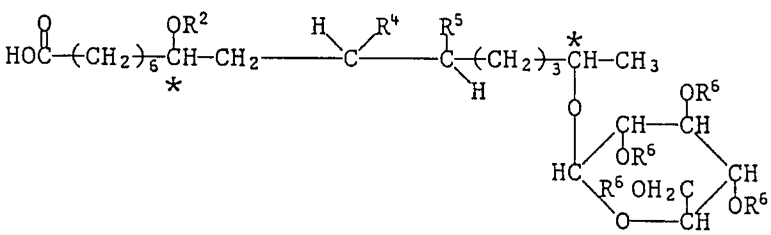

(IV)

oxidiert;

c) die Verbindung der Formel IV in einen zur Knüpfung einer Peptidbindung aktivierten Ester

überführt und den aktivierten Ester mit Laurin der Formel $H_2N{-}(CH_2){-}$ $SO_3H$ zu einer Verbindung der Formel V umsetzt,

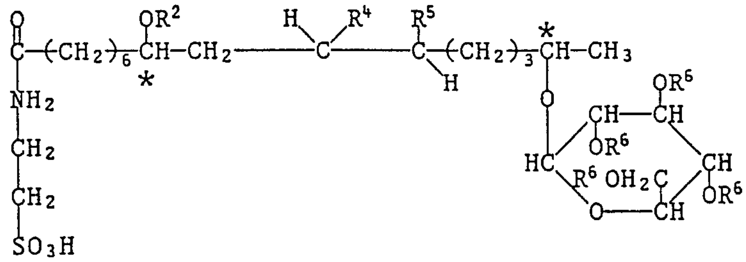
(V),

und darauf

d1) entweder aus den Verbindungen der Formel V, worin $R^4$ und $R^5$ je H bedeuten, die Schutzgruppen $R^2$ und $R^6$ abspaltet und durch H ersetzt; oder

d2) die Verbindungen der Formel V, worin $R^4$ und $R^5$ zusammen eine Bindung bedeuten, hydriert und gleichzeitig die Schutzgruppen $R^2$ und $R^6$ abspaltet und durch H ersetzt.

6. Verfahren gemäss Anspruch 5, dadurch gekennzeichnet, dass man bei der Verfahrensstufe a) die Reaktion in einem Halogenkohlenwasserstoff als Lösungsmittel und in Gegenwart von $BF_3$-Diethyletherat durchführt.

7. Verfahren gemäss Anspruch 5, dadurch gekennzeichnet, dass in Formel III $R^6$ von $R^2$ verschieden ist, und man $R^6$ abspaltet und danach eine zu $R^2$ identische Schutzgruppe einführt.

8. Verfahren gemäss Anspruch 7, dadurch gekennzeichnet, dass $R^2$ Benzyl und $R^6$ Pivaloyl sind.

9. Verfahren gemäss Anspruch 5, dadurch gekennzeichnet, dass man die Oxidation in der Verfahrensstufe b) mit einer Cer(IV)-Salz in Gegenwart von Wasser durchführt.

10. Verfahren gemäss Anspruch 5, dadurch gekennzeichnet, dass es sich bei dem aktivierten Ester der Verfahrensstufe c) um einen Ester mit einem N-Hydroxyimidrest in der Estergruppe handelt.

11. Verfahren gemäss Anspruch 5, dadurch gekennzeichnet, dass die Hydrierung und Abspaltung der Schutzgruppen bei der Verfahrensstufe d2) mit Wasserstoff in Gegenwart eines Katalysators erfolgt.

12. Verfahren gemäss Anspruch 5, dadurch gekennzeichnet, dass es sich bei den Schutzgruppen $R^1$, $R^2$ und $R^3$ in Formel II um Trialkylsilyl, ein mit $C_1$-$C_4$-Alkoxy substituiertes Phenyl, unsubstituiertes oder mit $C_1$-$C_4$-Alkyl substituiertes Benzyl, Diphenylmethyl oder Trityl, oder $C_1$-$C_8$-Acyl handelt.

13. Verfahren gemäss Anspruch 5, dadurch gekennzeichnet, dass $R^1$, $R^2$ und $R^3$ voneinander verschieden sind.

14. Verfahren gemäss Anspruch 13, dadurch gekennzeichnet, dass $R^1$ Trialkylsilyl, $R^2$ Benzyl, Diphenylmethyl oder Trityl bedeuten, und $R^3$ Alkoxy-substituiertes Phenyl ist oder die gleiche Bedeutung wie $R^2$ hat.

15. Verbindungen der Formel II

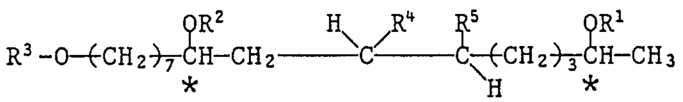
(II),

worin * die optischen Isomeren (R,R), (R,S), (S,R) und (S,S) bedeutet, $R^4$ und $R^5$ je für H oder beide zusammen für eine Bindung stehen und $R^1$, $R^2$ und $R^3$ unabhängig voneinander je eine Schutzgruppe bedeuten.

16. Verbindungen gemäss Anspruch 15, worin $R^1$, $R^2$ und $R^3$ Trialkylsilyl, ein mit $C_1$-$C_4$-Alkoxy substituiertes Phenyl, unsubstituiertes oder mit $C_1$-$C_4$-Alkyl substituiertes Benzyl, Diphenylmethyl oder Trityl, oder $C_1$-$C_8$-Acyl.

17. Verbindungen gemäss Anspruch 15, worin $R^1$, $R^2$ und $R^3$ voneinander verschieden sind.

18. Verbindungen gemäss Anspruch 17, worin $R^1$ Trialkylsilyl, $R^2$ Benzyl, Diphenylmethyl oder Trityl bedeuten, und $R^3$ Alkoxy-substituiertes Phenyl ist oder die gleiche Bedeutung wie $R^2$ hat.

19. Verbindungen gemäss Anspruch 15, worin $R^4$ und $R^5$ eine Bindung, $R^1$ t-Butyldimethylsilyl, $R^2$ Benzyl und $R^3$ p-Methoxyphenyl sind.

20. Verwendung der Verbindungen der Formel I zum Schützen von Kirschen vor dem Befall mit Kirschfliegen.

21. Verfahren zum Schützen von Kirschen vor dem Befall mit Kirschfliegen, dadurch gekennzeichnet, dass man eine markierend wirksame Menge der Verbindungen der Formel I auf die zu schützenden Kirschen appliziert.